# EUROPEAN PATENT APPLICATION

(11) **EP 4 335 437 A1**
(43) Date of publication of application: **13.03.2024**
(21) Application number: 22194419.2
(22) Date of filing: 07.09.2022
(51) Int. Cl.: A61K 31/37, A61P 37/02, C12N 5/00, C12N 5/0783

(54) **UROLITHINS AND T-CELL MEDIATED IMMUNE RESPONSE**

(71) Applicant: Chemotherapeutisches Forschungsinstitut Georg-Speyer-Haus, 60596 Frankfurt am Main (DE); Deutsches Krebsforschungszentrum Stiftung des öffentlichen Rechts, 69120 Heidelberg (DE)
(72) Inventor: GRETEN, Florian, 60596 Frankfurt am Main (DE); DENK, Dominic Pascal, 60528 Frankfurt am Main (DE); PETROCELLI, Valentina, 60322 Frankfurt am Main (DE); BUCHHOLZ, Christian, 60594 Frankfurt am Main (DE); BRAUN, Angela, 63225 Langen (DE)
(74) Representative: Kuttenkeuler, David

(57) **Abstract**

The invention is based on the use of Urolithin A for improving mitochondrial health in immune cells such as T-cells. The invention provides new strategies to enhance immune cell based therapies, such as adoptive T-cell therapy. The compounds and compositions of the invention are preferably useful for therapies that involve in vitro T cell modification and expansion, such as modification of T cells with a chimeric antigen receptor (CAR) for treating an antigen associated disorder, such as cancer.

## Description

### FIELD OF THE INVENTION

The invention is based on the use of Urolithin A for improving mitochondrial health in immune cells such as T-cells. The invention provides new strategies to enhance immune cell based therapies, such as adoptive T-cell therapy. The compounds and compositions of the invention are preferably useful for therapies that involve in vitro T cell modification and expansion, such as modification of T cells with a chimeric antigen receptor (CAR) for treating an antigen associated disorder, such as cancer.

### DESCRIPTION

Colorectal cancer (CRC) is the third-most diagnosed cancer worldwide, and its mortality remains high, especially in advanced stages (Sung et al., 2021). Colorectal cancer progression strongly depends on its complex tumor microenvironment (TME) (Schmitt and Greten, 2021) and the particular importance of T cells for CRC prognosis is well exemplified by the development of an immmunoscore (Pages et al., 2018). Importantly, the local immunoscore was shown to predict patient survival in CRC patients with metastases (van den Eynde et al., 2018). However, CRC comprises a clear unresolved paradox as only microsatellite instable (MSI+) colorectal cancers have been demonstrated to adequately respond to immune checkpoint blockade so far (Le et al., 2015). Successful strategies to sensitize the large majority of microsatellite stable (MSS) colorectal tumors to immune therapy are lacking. Recently, the inventors have shown that mitophagy in intestinal epithelial cells (IEC) in mice with a IECrestricted Stat3 deletion (Stat3^{ΔIEC}) increases antigen presentation thereby enhancing CD8+ T cell dependent anti-tumor immunity during early intestinal tumorigenesis (Ziegler et al., 2018). However, apart from proper antigen presentation, efficacy of immunotherapy is limited by T cell exclusion or T cell exhaustion (Chen and Mellman, 2017). The TME confers T cell exhaustion by structurally and metabolically modifying T cells via chronic signalling cues (Scharping et al., 2016). Among many pathways driving changes in T cell phenotype and function, mitochondrial dynamics have been described to alter CD8+ T cell fate (Buck et al., 2016). Of note, effector tumor-infiltrating lymphocytes (TIL) in the TME display decreased mitophagy, driving terminal exhaustion by accumulation of depolarized mitochondria (Yu et al., 2020b). Therefore, enhancing T cell fitness in the TME by promoting mitophagy represents an attractive goal for cancer therapy, considering the potentially positive effect of mitophagy in both IEC and T cells. However, currently, no clinically-feasible methods of inducing mitophagy for cancer treatment are available.

Urolithin A (UA) is a natural metabolite of elagatinnines (ETs), of which pomegranates present a main source (Espín et al., 2013). Metabolisation into UA depends on the appropriate gut microbiome that undergoes alteration with age, limiting production of UA from ETs to less than half of the aging population (Cortés-Martín et al., 2018). Concentrated forms of UA demonstrated that dietary supplementation induces mitophagy in vivo, favorably affecting the progression of aging-related diseases (reviewed in(D'Amico et al., 2021)). Rodents fed with a UA-high diet display superior muscle function and recovery of muscle function in mice with Duchenne muscular dystrophy, whereas in C.elegans, UA conferns higher mitochondrial content and prolonged lifespan (Luan et al., 2021; Ryu et al., 2016). Moreover, UA has immunomodulatory effects in monocytic cells attenuating inflammation in various tissues (Toney et al., 2021) and an impact on adaptive immunity by expanding FoxP3+ T regulatory cells (Ghosh et al., 2021) or blocking Th1/Th17 cell infiltration in a model of EAE (Shen et al., 2021). In humans, commercially-available supplements containing UA have been deemed safe with a favorable bioavailability profile (Andreux et al., 2019). Here the inventors explored the effects of UA in colorectal carcinogenesis and demonstrate that UA induces MHC-I upregulation on tumor epithelia and promotes expansion of T memory stem cells (TSCM), thus strongly inducing a protective anti-tumor CD8+ T cell immunity.

Thus, it is an object of the invention to provide methods for enhancing T cell health for improving adoptive immune cell therapies for the treatment of various disorders.

### BRIEF DESCRIPTION OF THE INVENTION

Generally, and by way of brief description, the main aspects of the present invention can be described as follows:
In **a first aspect,** the invention pertains to a compound for use in a therapeutic modulation of a T-cell mediated immune response in a subject, wherein the compound is a mitophagy agonist, preferably selected from a compound of formula (I), 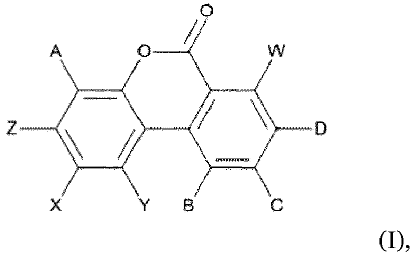 wherein A, B, C, D, X, Y, and W are independently selected from H or OH, or a salt, isomer, tautomer, prodrug thereof.

In a **second aspect**, the invention pertains to an *ex-vivo* method for modulating a T-cell, the method comprising:
(a) Providing a T-cell;
(b) Contacting the T-cell with an mitophagy agonist, preferably a compound of formula (I), 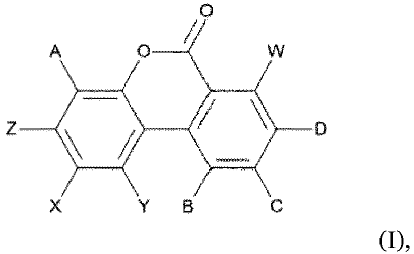 wherein A, B, C, D, X, Y, and W are independently selected from H or OH, or a salt, isomer, tautomer, or prodrug thereof,
   for a time sufficient to induce mitophagy in the T-cell, and thereby obtain a modulated T-cell;
(c) Optionally, further genetically modifying the modulated T-cell;
(d) Optionally, further culturing and / or proliferating the modulated T-cell.

In **a third aspect,** the invention pertains to T cell or cellular composition comprising T-cells, which are obtainable by, or obtained by, a method of the first or second aspect.

In **a fourth aspect,** the invention pertains to a cellular composition for use in the treatment of a disease in a subject, wherein the treatment comprises an adoptive T-cell transfer with the steps of
(a) Obtaining a cellular sample of the subject containing T-cells;
(b) Optionally purifying and/or culturing (expanding) the T-cells from the cellular sample;
(c) Contacting the T-cell with an mitophagy agonist, preferably a compound of formula (I), 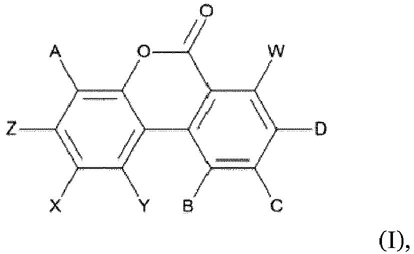
(d) wherein A, B, C, D, X, Y, and W are independently selected from H or OH, or a salt, isomer, tautomer, or prodrug thereof,
(e) for a time sufficient to induce mitophagy in the T-cell, and thereby obtain modulated T-cells;
(f) Optionally, further genetically modifying the modulated T-cells;
(g) Optionally, further culturing and / or proliferating the modulated T-cells.
(h) Administrating the modulated T-cells to the patient and thereby treating the disease.

In **a fifth aspect,** the invention pertains to a method for modulating a T-cell, the method comprising the steps of contacting a T-cell with a mitophagy agonist for a time sufficient to induce mitophagy in the T-cell, preferably wherein the mitophagy agonist is a compound recited in the first aspect.

In **a sixth aspect,** the invention pertains to a method for treating a subject suffering from a disease that is treatable by enhancing a T-cell mediated immune response, the method comprising:
(a) Administering to the subject a therapeutically effective amount of a mitophay agonist, preferably selected from a compound recited in claim 1; or
(b) Modulating a T-cell ex-vivo with a mitophagy agonist to obtain a modulated T-cell, and administering to the subject with a therapeutically effective amount of the modulated T-cell.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

As used herein, the term "antigen" is a molecule capable of being bound by an antibody or T-cell receptor. An antigen is additionally capable of inducing a humoral immune response and/or cellular immune response leading to the production of B and/or T lymphocytes. As used herein, the term "patient" or "subject" refers to a living mammalian organism, such as a human, monkey, cow, sheep, goat, dog, cat, mouse, rat, guinea pig, or transgenic species thereof. In certain embodiments, the patient or subject is a primate. Non limiting examples of human patients are adults, juveniles, infants and fetuses.

"Treating" or treatment of a disease or condition refers to executing a protocol, which may include administering one or more drugs to a patient, in an effort to alleviate signs or symptoms of the disease. Desirable effects of treatment include decreasing the rate of disease progression, ameliorating or palliating the disease state, and remission or improved prognosis. Alleviation can occur prior to signs or symptoms of the disease or condition appearing, as well as after their appearance. Thus, "treating" or "treatment" may include "preventing" or "prevention" of disease or undesirable condition. In addition, "treating" or "treatment" does not require complete alleviation of signs or symptoms, does not require a cure, and specifically includes protocols that have only a marginal effect on the patient.

The term "effective," as that term is used in the specification and/or claims, means adequate to accomplish a desired, expected, or intended result. "Effective amount," "therapeutically effective amount," or "pharmaceutically effective amount" when used in the context of treating a patient or subject with a compound means that amount of the compound which, when administered to a subject or patient for treating or preventing a disease, is an amount sufficient to effect such treatment or prevention of the disease.

"Prevention" or "preventing" includes: (1) inhibiting the onset of a disease in a subject or patient which may be at risk and/or predisposed to the disease but does not yet experience or display any or all of the pathology or symptomatology of the disease, and/or (2) slowing the onset of the pathology or symptomatology of a disease in a subject or patient which may be at risk and/or predisposed to the disease but does not yet experience or display any or all of the pathology or symptomatology of the disease.

As generally used herein "pharmaceutically acceptable" refers to those compounds, materials, compositions, and/or dosage forms which are, within the scope of sound medical judgment, suitable for use in contact with the tissues, organs, and/or bodily fluids of human beings and animals without excessive toxicity, irritation, allergic response, or other problems or complications commensurate with a reasonable benefit/risk ratio.

In **a first aspect,** the invention pertains to a compound for use in a therapeutic modulation of a T-cell mediated immune response in a subject, wherein the compound is a mitophagy agonist, preferably selected from a compound of formula (I), 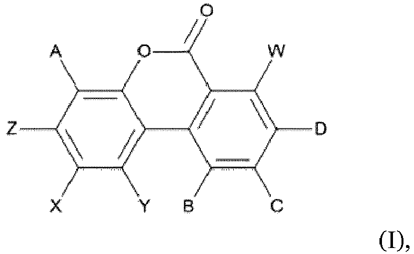 wherein A, B, C, D, X, Y, and W are independently selected from H or OH, or a salt, isomer, tautomer, prodrug thereof.

In preferred embodiments of the invention, the compound is preferably selected from urolithin A, urolithin B, urolithin C and urolithin D, but most preferably is urolithin A, or a salt, isomer, tautomer, prodrug thereof.

In another embodiment of the invention, the therapeutic modulation is a therapy or cotherapy of a proliferative disease in the subject.

In a preferred embodiment, the modulation of a T-cell mediated immune response involves any one or a combination of:
an induction of mitophagy in the T-cell;
an increase in T-memory stem cells (T_{SCM});
an increase in naive like T-cells (T_{N});
an increase in T-cell mediated anti-tumor immunity;
increase in T-cell WNT signaling;
increased T-cell mitochondrial health.

The method of the invention may comprise an administration of the compound to the subject, or comprises an administration of T-cells contacted with the compound to the subject. For example, the T-cells contacted with the compound are autologous T-cells obtained from the subject, subsequently brought into contact with the compound, optionally, expanded and/or genetically modified, and reinfused to the patient.

In one embodiment, provided herein are methods of providing a T-cell response in a human subject having a disease, the method comprising (a) obtaining a sample of cells from a subject, the sample comprising T cells; (b) culturing the sample of cells ex vivo in a medium that selectively enhances health of tumor antigen-specific T cells by the presence of the compound of the invention in the medium, and (c) administering an effective amount of the tumor antigen-specific T cells to the subject to provide a T cell response.

In one preferred embodiment of the invention, the treatment therefor involves an adoptive cell therapy, such as an adoptive T-cell therapy, preferably wherein the T-cells are modified for example with a Chimeric Antigen Receptor (CAR). In some aspects, the methods further comprise integrating a DNA encoding a chimeric antigen receptor (CAR) into the genome of the cells, to provide a population of transgenic CAR-expressing T cells. In some aspects, the methods further comprise integrating a DNA encoding a T-cell receptor into the genome of the cells, to provide a population of transgenic T- cell receptor-expressing T cells.

Certain aspects of the present invention provide a cell-based therapy for treating diseases, such as tumors. Tumors for which the present treatment methods are useful include any malignant cell type, such as those found in a solid tumor or a hematological tumor. Exemplary solid tumors can include, but are not limited to, a tumor of an organ selected from the group consisting of pancreas, colon, cecum, stomach, brain, head, neck, ovary, kidney, larynx, sarcoma, lung, bladder, melanoma, prostate, and breast. Exemplary hematological tumors include tumors of the bone marrow, T or B cell malignancies, leukemias, lymphomas, blastomas, myelomas, and the like. Further examples of cancers that may be treated using the methods provided herein include, but are not limited to, carcinoma, lymphoma, blastoma, sarcoma, leukemia, squamous cell cancer, lung cancer (including small-cell lung cancer, nonsmall cell lung cancer, adenocarcinoma of the lung, and squamous carcinoma of the lung), cancer of the peritoneum, hepatocellular cancer, gastric or stomach cancer (including gastrointestinal cancer and gastrointestinal stromal cancer), pancreatic cancer, glioblastoma, cervical cancer, ovarian cancer, liver cancer, bladder cancer, breast cancer, colon cancer, colorectal cancer, endometrial or uterine carcinoma, salivary gland carcinoma, kidney or renal cancer, prostate cancer, vulval cancer, thyroid cancer, various types of head and neck cancer, melanoma, superficial spreading melanoma, lentigo malignant melanoma, acral lentiginous melanomas, nodular melanomas, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's macroglobulinemia), chronic lymphocytic leukemia (CLL), acute lymphoblastic leukemia (ALL), Hairy cell leukemia, multiple myeloma, acute myeloid leukemia (AML) and chronic myeloblastic leukemia.

The terms "contacted" and "exposed," when applied to a cell, are used herein to describe the process by which a compound of the invention (of formula I and preferably urolithin A) are placed in direct juxtaposition with the target cell, for example in a cell culture medium in vitro. In alternative embodiments this may be practiced in vivo.

A cell-based therapy may be administered before, during, after, or in various combinations relative to a second treatment. The administrations may be in intervals ranging from concurrently to minutes to days to weeks. In embodiments where the cell-based therapy is provided to a patient separately from a second agent, one would generally ensure that a significant period of time did not expire between the time of each delivery, such that the two treatments would still be able to exert an advantageously combined effect on the patient. In such instances, it is contemplated that one may provide a patient with the cell-based therapy and the second therapy within about 12 to 24 or 72 h of each other and, more particularly, within about 6-12 h of each other. In some situations it may be desirable to extend the time period for treatment significantly where several days (2, 3, 4, 5, 6, or 7) to several weeks (1, 2, 3, 4, 5, 6, 7, or 8) lapse between respective administrations.

In **a second aspect,** the invention pertains to an *ex-vivo* method for modulating a T-cell, the method comprising:
(a) Providing a T-cell;
(b) Contacting the T-cell with an mitophagy agonist, preferably a compound of formula (I), 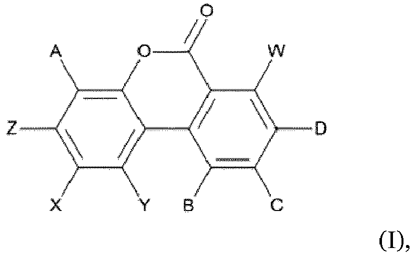 wherein A, B, C, D, X, Y, and W are independently selected from H or OH, or a salt, isomer, tautomer, or prodrug thereof,
   for a time sufficient to induce mitophagy in the T-cell, and thereby obtain a modulated T-cell;
(c) Optionally, further genetically modifying the modulated T-cell;
(d) Optionally, further culturing and / or proliferating the modulated T-cell.

In one preferred embodiment, in step b, the T-cell is modulated into a T-memory stem cells (T_{SCM}) or a naive like T-cell (T_{N}).

Step (c) may be performed before and/or after the contacting in step (b) in accordance with the invention. Between any step, a culturing, selection and/or expanding of T-cells may be introduced. Also possible is a treatment of the T-cells with the compound in accordance with the invention and an optional expansion, then a modulation of the T-cells, for example with a CAR construct, and a following second treatment of the cells with the compound of the invention. In addition, in a further embodiment, the T-cells obtained from a patient are first genetically modified (for example with a CAR construct), and subsequently treated with the compound in accordance with the invention.

In another preferred embodiment, step c includes genetically introducing a DNA construct for enhancing an immune response mediated by the T-cell. Such methods are for example also described herein above for the first aspect and are equally applicable for the other aspects of the invention.

A construct, such as a DNA construct, may be selected from an expression construct expressing a T cell receptor (TCR), or a TCR-like protein (such as a scTCR, soluble TCR etc), an antibody, or anti-body like protein, a chimeric antigen receptor (CAR), an immune receptor or ligand, such as PD1, PDL1, CD28, or a derivative thereof; and/or involves contacting the T-cell with a T-cell activator, such as an agonist of CD3/CD28, such as an antibody, and/or a T-cell activating cytokine.

Preferably, the T-cell is provided from a cellular sample of a subject suffering from a disease treatable by T-cell activation, such as a proliferative disease (cancer) or an infectious disease.

In another embodiment, the T-cell after modification is proliferated and expanded to obtain a clinical grade T-cell preparation suitable for using in adoptive T-cell therapy. Such

In **a third aspect,** the invention pertains to T cell or cellular composition comprising T-cells, which are obtainable by, or obtained by, a method of the first or second aspect.

In **a fourth aspect,** the invention pertains to a cellular composition for use in the treatment of a disease in a subject, wherein the treatment comprises an adoptive T-cell transfer with the steps of
(a) Obtaining a cellular sample of the subject containing T-cells;
(b) Optionally purifying and/or culturing (expanding) the T-cells from the cellular sample;
(c) Contacting the T-cell with an mitophagy agonist, preferably a compound of formula (I), 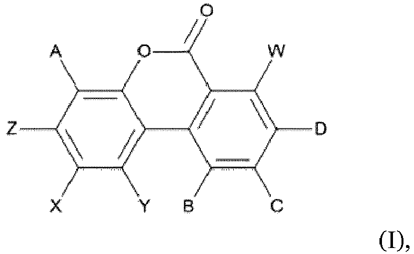
(d) wherein A, B, C, D, X, Y, and W are independently selected from H or OH, or a salt, isomer, tautomer, or prodrug thereof,
(e) for a time sufficient to induce mitophagy in the T-cell, and thereby obtain modulated T-cells;
(f) Optionally, further genetically modifying the modulated T-cells;
(g) Optionally, further culturing and / or proliferating the modulated T-cells.
(h) Administrating the modulated T-cells to the patient and thereby treating the disease.

Preferably, in step **c**, the T-cell is modulated into a T-memory stem cells (T_{SCM}) or a naive like T-cell (T_{N}).

In further embodiments, step **d** includes genetically introducing a construct for enhancing an immune response mediated by the T-cell. Such construct is selected from a T cell receptor (TCR), or a TCR-like protein (such as a scTCR, soluble TCR etc), an antibody, or antibody like protein, a chimeric antigen receptor (CAR), an immune receptor or ligand, such as PD1, PDL1, CD28, or a derivative thereof.

Yet in other embodiments, T-cell is provided from a cellular sample of a subject suffering from a disease treatable by T-cell activation, such as a proliferative disease (cancer) or an infectious disease. Such a T-cell after modification may be proliferated and expanded to obtain a clinical grade T-cell preparation suitable for using in adoptive T-cell therapy.

In **a fifth aspect,** the invention pertains to a method for modulating a T-cell, the method comprising the steps of contacting a T-cell with a mitophagy agonist for a time sufficient to induce mitophagy in the T-cell, preferably wherein the mitophagy agonist is a compound recited in the first aspect.

In **a sixth aspect,** the invention pertains to a method for treating a subject suffering from a disease that is treatable by enhancing a T-cell mediated immune response, the method comprising:
(a) Administering to the subject a therapeutically effective amount of a mitophay agonist, preferably selected from a compound recited in claim 1; or
(b) Modulating a T-cell ex-vivo with a mitophagy agonist to obtain a modulated T-cell, and administering to the subject with a therapeutically effective amount of the modulated T-cell.

In another aspect of the invention, a compound as defined in the first aspect, and preferably Urolithin A, is for use in the treatment of a disease, wherein the disease is characterized by being responsive by a T-cell mediated immune response, such as a proliferative disease as defined herein elsewhere (in particular cancer). Preferably such a cancer is characterized by eliciting in the patient a T-cell mediated immune response against cells the cells of the cancer, and wherein a treatment of T-cells specific for the cancer cells are brought into contact with the compound in-vitro or in-vivo. For applications in vitro, the T-cells specific for the cells associated with the disease (such as the cancer cells) are treated ex-vivo with the compound (for example in order to increase T-cell health) and then administrated to the patient in need of the treatment. Such T-cells may be autologous to the patient. Alternatively, the compound may be administered to the patient directly in order to contact T-cells of the patient with the compound in vivo (for example in order to increase T-cell health in the patient).

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:

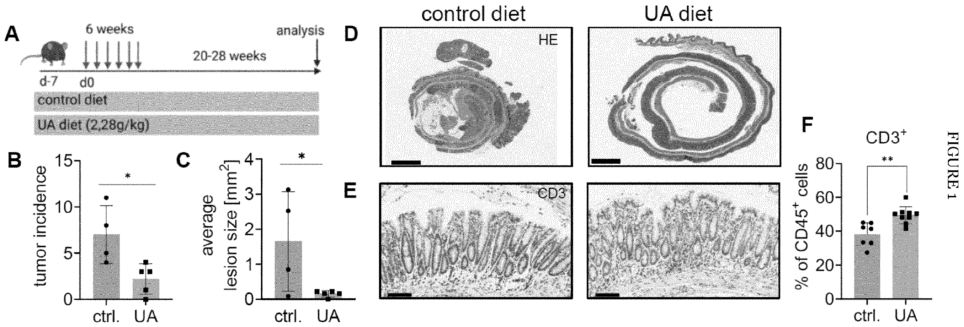

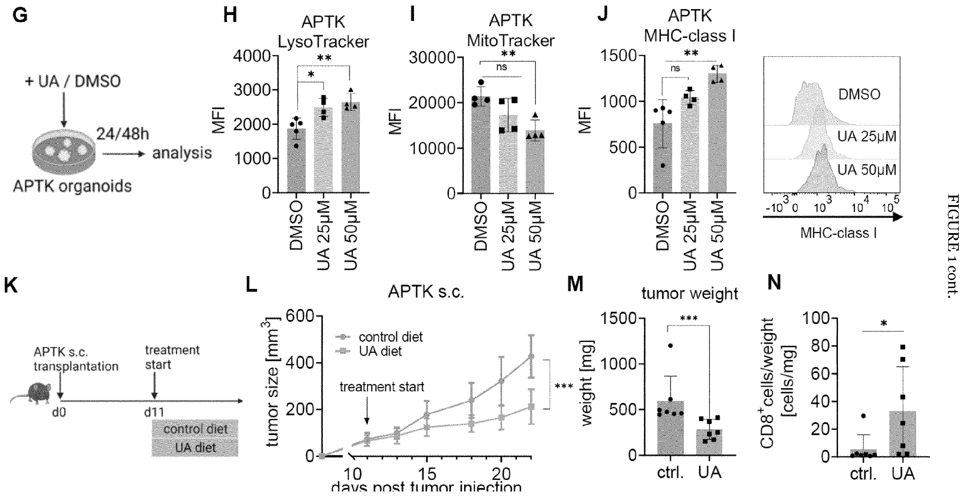

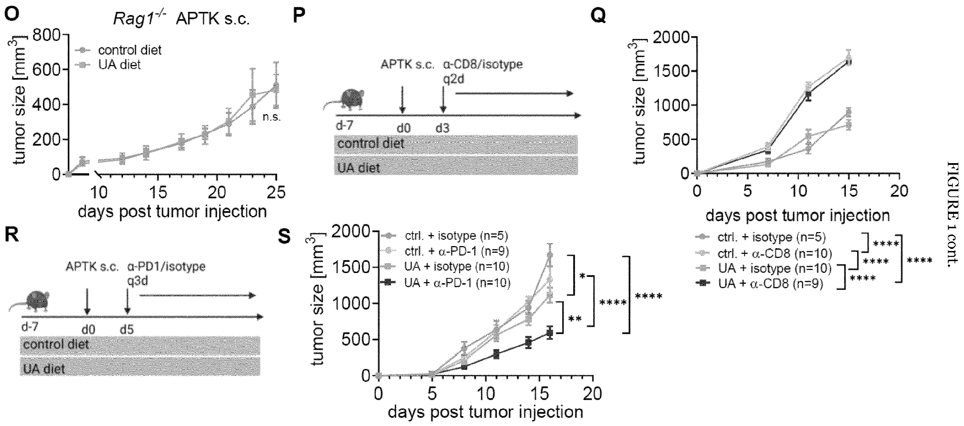

**Figure 1****: Oral UA confers anti-tumor effects in murine colorectal cancer in a T-cell dependent manner (A)** Schematic representation of AOM model and treatment regimen. (**B-C**) Tumor incidence and average lesion size of AOM-induced tumors receiving a UA-containing diet or control diet. Data are mean ± SD, n=4/5, ^{∗}p < 0.05 by two-sided t-test. One of two independent experiments shown. **(D)** Representative images of swiss-roll sections from AOM-induced colon tumors. Scale bar = 2mm. **(E)** Representative images of CD3⁺ T cell staining of colons from AOM-treated mice as depicted in (A, D). Scale bar = 100µm. **(F)** Relative number of CD45⁺CD3⁺ T cells in colons of AOM-treated mice at week 24, Data are mean ± SD, n=7/8, ^{∗∗}p < 0.01 by two-sided t-test **(G)** Organoid treatment scheme. APTK-organoids were incubated in the presence of UA or DMSO for the indicated time points, prior to flow cytometric analysis. **(H)** Quantification of lysosome formation as assessed by lysotracker MFI via flow cytometry after 24h incubation at the presence of various UA concentrations. Data are mean ± SD, n=5/4/4, ^{∗}p < 0.05, ^{∗∗}p < 0.01 by one-way ANOVA followed by Tukey's multiple comparison test. Representative results from one of two indepdent experiments are shown. **(I)** Mitotracker signal of APTK organoids incubated for 24h at the presence of UA in *vitro* (n=4/4/4) and antigen presentation via MHC-I molecules (J) after 48h of treatment (n=5/4/4). Data are mean ± SD, ^{∗∗}p < 0.01 by one-way ANOVA followed by Tukey's multiple comparison test. Results from one of two independent experiments are shown. **(K)** Experimental setup of oral UA administration in mice with established APTK-s.c. tumors. Treatment diet was initiated eleven days after tumor injection and maintained until the end of the experiment. **(L)** Growth curve of mice subcutaneously transplanted with APTK tumors, receiving either UA-containing or control food. Data are mean ± SD, n=7, ^{∗∗∗}p <0.001 by two-sided t-test. One out of two independent experiments are shown. **(M)** End point tumor weight, **(N)** CD8⁺ T cell infiltration in APTK-s.c. tumors assessed by flow cytometry, normalizing total number of CD8⁺ T cells to tumor weight. Data are mean ± SD, n=7, ^{∗}p < 0.05 and ^{∗∗∗}p <0.001 by Mann-Whitney test. **(O)** Size of subcutaneous APTK tumors in *Rag1*^{*-*/*-*} mice receiving UA-containing or control food. Treatment was initiated as depicted in **(K)**. Data are mean ± SD, n=7, depicting one out of two independent experiments. **(P)** CD8⁺ T cell depleting or isotype control antibodies were applied every two days starting three days after s.c. injection of APTK organoids in C57BL/6 mice. **(Q)** Effect of CD8⁺ T cell depletion on size of subcutaneous APTK tumors in C57BL/6 mice receiving UA-containing or control food. Data are mean ± SEM, isotype n=5, UA food + a-CD8 n=9, other groups n=10, ^{∗∗∗∗}p < 0.0001 by one-way ANOVA followed by Tukey's multiple comparison test. Data shown represents pooled data from two experiments with comparable results. **(R)** a-PD-1 or isotype antibodies were injected every three days starting five days after s.c. injection of APTK organoids in C57BL/6 mice. **(S)** Effect of a-PD-1 treatment on size of subcutaneous APTK tumors in C57BL/6 mice receiving UA-containing or control food. Data are mean ± SEM, isotype n=5; control food + a-PD-1 n=9; other groups n=10; ^{∗}p < 0.05, ^{∗∗}p < 0.01 and ^{∗∗∗∗}p <0.0001 by one-way ANOVA followed by Tukey's multiple comparison test. Data shown represents pooled data from two experiments with comparable results.

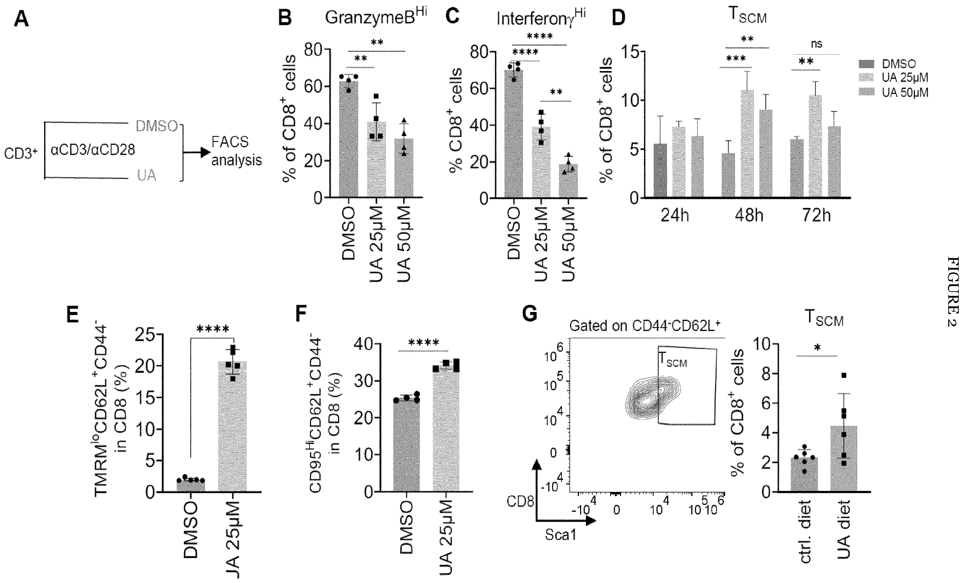

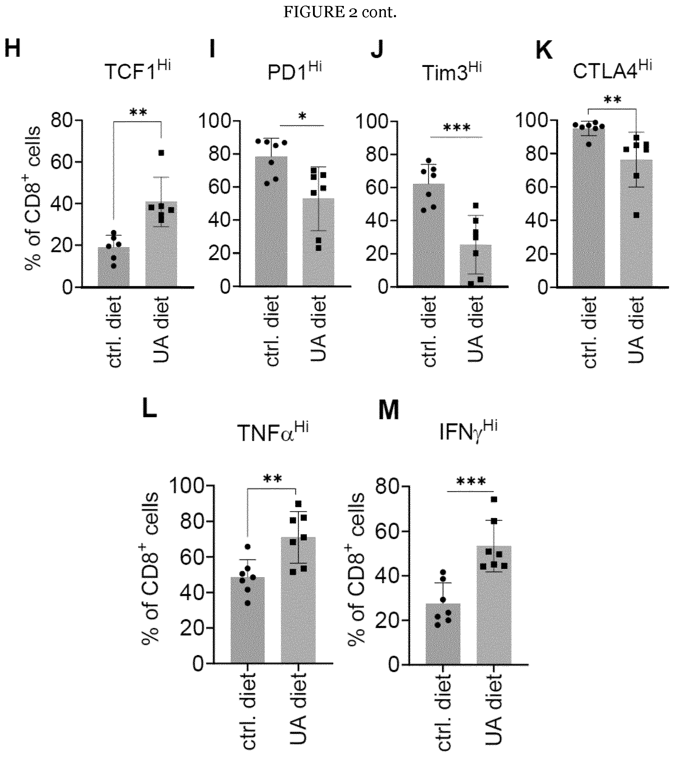

**Figure 2****: UA promotes T_{SCM} cell expansion (A)** Scheme of CD3⁺ T cell activation, treatment and analysis. (**B, C**) Granzyme B and IFNg expression in aCD3/aCD28 stimulated CD3⁺ T cells in the presence of UA or DMSO after 48h. Data are mean ± SD, n=₄, ^{∗∗}p < 0.01 and ^{∗∗∗∗}p < 0.0001 by one-way ANOVA followed by Tukey's multiple comparison test. **(D)** Quantification of CD44⁻CD62L⁺Sca1^{Hi} T_{SCM} 24h, 48h and 72h after aCD3/aCD28 stimulation. Data are mean ± SD, n=4 from four independent experiments, ^{∗∗}p < 0.01; p^{∗∗∗} <0.001 by two-way ANOVA followed by Tukey's multiple comparison test. **(E)** Frequency of CD62L⁺CD44⁻CD8⁺ T cells with low mitochondrial membrane potential (TMRM^{Lo}) after 48h in the presence or absence of UA (25µM). Data are mean ± SD, n=5; ^{∗∗∗∗}p <0.0001 by two-sided t-test. **(F)** Frequency of CD95^{Hi}CD62L⁺CD44⁻CD8⁺ T cells 48hours after in aCD3/aCD28 stimulation in the presence or absence of UA (25µM). Data are mean ± SD; n=5; ^{∗∗∗∗}p < 0,0001 by two-sided t-test. **(G)** Frequency of T_{SCM} (n=7), **(H)** TCF1 (n=7), **(I)** PD1^{Hi} (n=6), **(J)** Tim3^{Hi} (n=6) and **(K)** CTLA4^{Hi} (n=6) within CD8⁺ TIL of APTK-induced tumors receiving control or UA-containing diet. Data are mean ± SD, ^{∗}p < 0.05; ^{∗∗}p < 0,01; ^{∗∗∗}p <0,001 by two-sided t-test. **(L)** TNFa and **(M)** IFNg expression in CD8⁺ TIL of APTK-induced tumors receiving control or UA-containing diet upon re-stimualtion with PMA/ionomycin. Data are mean ± SD, n=7 per group, ^{∗∗}p < 0.01 and ^{∗∗∗}p <0,001 by two-sided t-test.

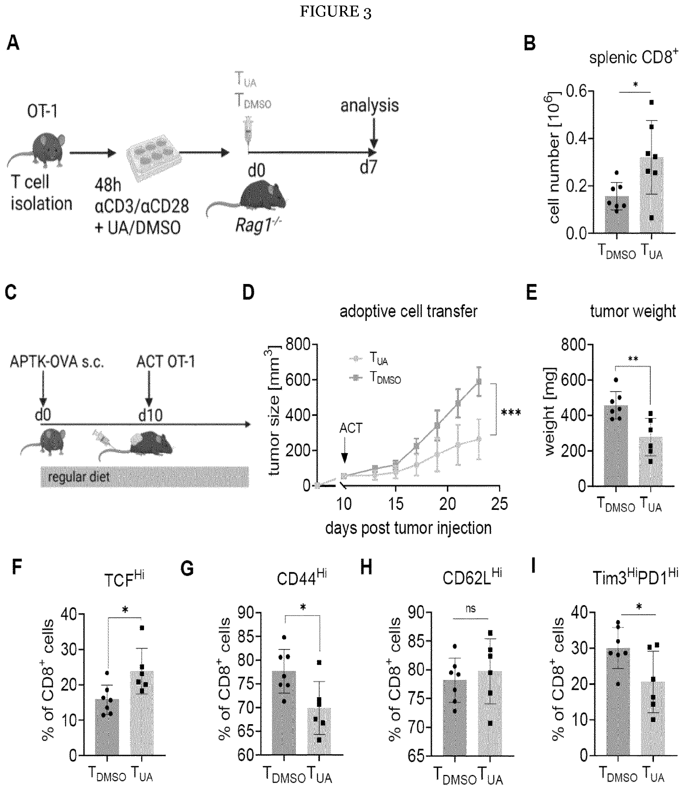

**Figure 3****: UA treatment augments efficacy of adoptive cell transfer** (A) Experimental setup of adoptive cell transfer into *Rag1*^{*-*/*-*} mice: CD3⁺ T cells from OT-i donor mice were stimulated for 48h with aCD3/aCD28 in the presence of UA (25µM; T_{UA}) or DMSO (T_{DMSO}) prior to transfer. **(B)** Number of splenic CD8⁺ T cells in *Rag1*^{*-*/*-*} mice one week after adoptive transfer of either UA-treated or control T cells. Data are mean ± SD with n=7 per group, ^{∗}p < 0.05 by two-sided t-test. **(C)** Experimental setup of adoptive cell transfer of OT-i CD3⁺ T cells in mice bearing APTK-OVA s.c. tumors. *Ex vivo* stimulation prior to transfer was performed as depicted in (A). **(D)** Growth curve of s.c. APTK-OVA tumors treated as depicted in (C), UA: n=6, DMSO: n=7, Data are mean ± SD, ^{∗∗∗}p <0,001 by two-sided t-test. **(E)** Final tumor weight of s.c. transplanted APTK-OVA tumors in *Rag1*^{*-*/*-*} mice transplanted with T_{UA} or T_{DMSO}. Data are mean ± SD, ^{∗∗}p < 0,01 by two-sided t-test. **(F-I)** Analysis of TIL of ACT-treated *Rag1*^{*-*/*-*} mice carrying APTK-OVA tumors: **(F)** Expression of CD44, **(G)** TCF1 and **(H)** CD62L in CD8⁺ TIL from APTK-OVA induced tumors that had received UA-treated OT-I T cells (n=6) or DMSO treated OT-I T cells (n=7), Data are mean ± SD, ^{∗}p < 0.05 by two-sided t-test, n.s. not significant. **(I)** Frequency of exhausted Tim3^{Hi}PD-1^{Hi} CD8⁺ TIL from APTK-OVA induced tumors that had received UA-treated OT-I T cells (n=6) or DMSO treated OT-I T cells (n=7). Data are mean ± SD, ^{∗}p < 0.05 by two-sided t-test.

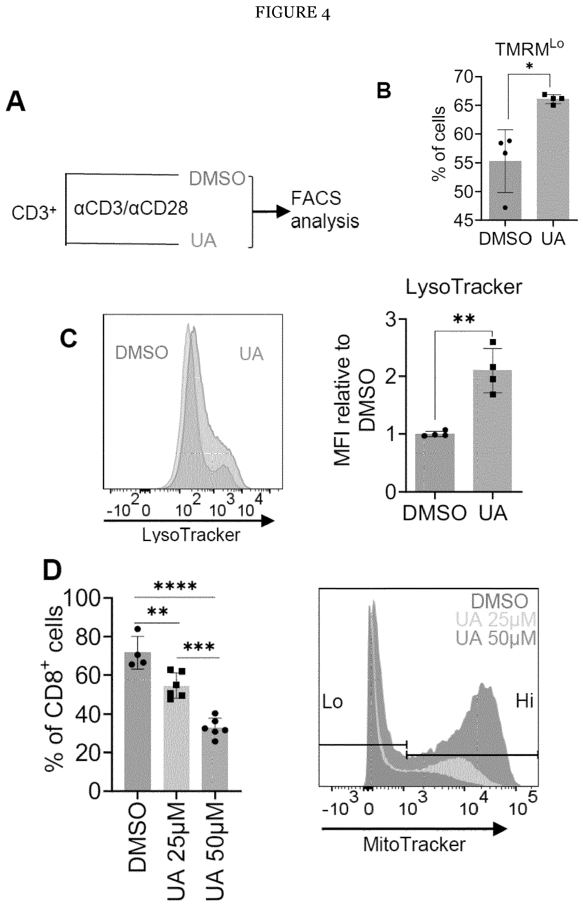

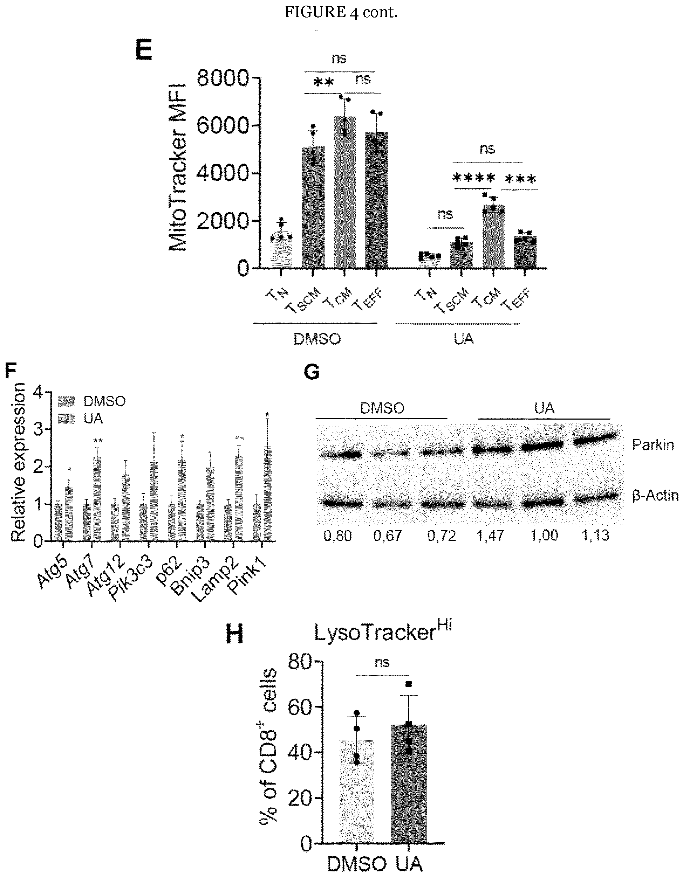

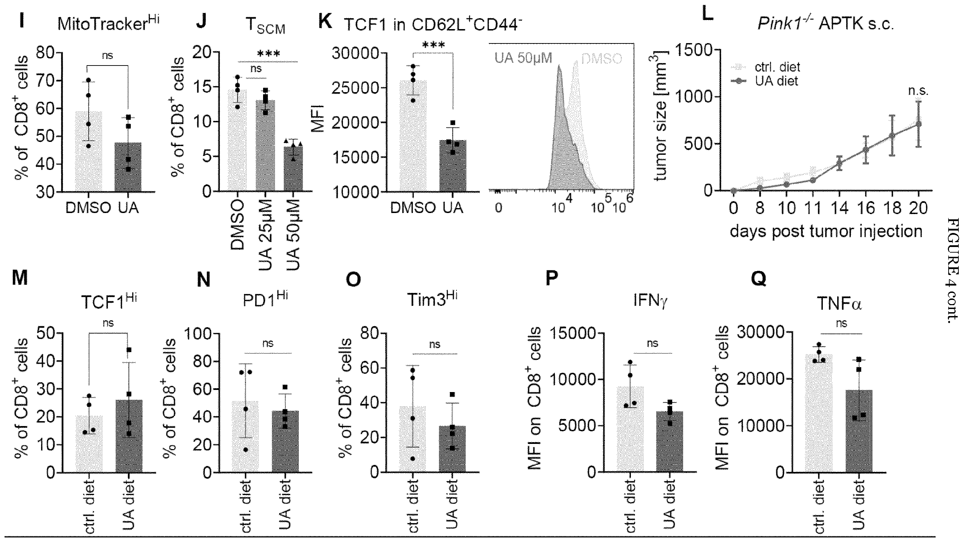

**Figure 4****: Urolithin A triggers Pinki-dependent mitophagy in T cells (A)** Scheme of CD3⁺ T cell activation, treatment and analysis. **(B)** Frequency of CD8⁺ T cells with low mitochondrial membrane potential (TMRM^{Lo}) after six hour stimulation with aCD3/aCD28 in the absence or presence of UA (50µM). Data are mean ± SD, n=4; ^{∗}p <0.05 by two-sided t-test. Data shown represents one of two independent experiments. **(C)** Quantification of lysosome formation in CD8⁺ T cells after 6h stimulation with aCD3/aCD28 in the absence or presence of UA (50µM). Data are mean ± SD, n=4; ^{∗∗}p <0.01 by two-sided t-test. Data shown represents one of two independent experiments. **(D)** Frequency of MitoTracker Red staining of CD8⁺ T cells after 24h stimulation with aCD3/aCD28 in the absence or presence of UA. Data are mean ± SD, n=6 (UA) or n=4 (DMSO), ^{∗∗}p < 0.01, ^{∗∗∗}p <0.001, ^{∗∗∗∗}p < 0.0001 by one-way ANOVA followed by Tukey's multiple comparison test. **(E)** MitoTracker expression in T cell subsets 24h after stimulation with aCD3/aCD28 in the absence or presence of UA. Data are mean ± SD, n=5, ^{∗∗}p < 0.01, ^{∗∗∗}p <0.001, ^{∗∗∗∗}p < 0.0001 by one-way ANOVA followed by Tukey's multiple comparison test. **(F)** qPCR analysis of various autophagy/mitophagy associated genes in T cells 24h after stimulation with aCD3/aCD28 in the absence or presence of UA (50µM), Data are ± SEM, n=3; ^{∗}p < 0.05 ,^{∗∗}p < 0.01by two-sided t-test. **(G)** Immunoblot analysis of indicated proteins in T cells 24h after stimulation with aCD3/aCD28 in the absence or presence of UA (50µM). **(H)** Quantification of lysosome formation in *Pink1*^{*-*/*-*} CD8⁺ T cells after 6h stimulation with aCD3/aCD28 in the absence or presence of UA (50µM). Data are mean ± SD, n=₄, n.s., not significant by two-sided t-test. Data shown represents one of two independent experiments. **(I)** Frequency of Mitotracker^{Hi} *Pink1*^{*-*/*-*} CD8⁺ T cells after 48h stimulation with aCD3/aCD28 in the absence or presence of UA (50µM). Data are mean ± SD, n=₄, n.s. not significant by two-sided t-test. Data shown represents one of two independent experiments. **(J)** Frequency of T_{SCM} in *Pink1*^{*-*/*-*} CD8⁺ T cells after 48h stimulation with aCD3/aCD28 in the absence or presence of UA (25 and 50µM), Data are mean ± SD, n=₄, ^{∗∗∗}p <0.001 by two-sided t-test. **(K)** Expression of TCF1 in CD62L⁺CD44⁻CD8⁺ cells from *Pink1*^{*-*/-}mice after 48h stimulation with aCD3/aCD28 in the absence or presence of UA (50µM), Data are mean ± SD, n=₄, ^{∗∗∗}p <0.001 by two-sided t-test. **(L)** Growth curve of *Pink1*^{*-*/*-*} mice s.c. injected with APTK-organoids, fed UA-containing or control diet as depicted in Fig.1K. Data are mean ± SD, n=4/group. **(M)** Frequency of TCF^{Hi}, **(N)** PD^{Hi}, **(O)** Tim3^{Hi} CD8⁺ TIL in APTK-induced tumors in *Pink1*^{*-*/*-*} mice fed either UA or control diet. Data are mean ± SD, n=4/group. **(P)** Expression of IFNγ and **(Q)** TNFα in CD8⁺ TIL from *Pink1*^{*-*/*-*} restimulated *ex vivo* with PMA/Ionomycin in the presence of Brefeldin A for three hours. Data are mean ± SD, n=4.

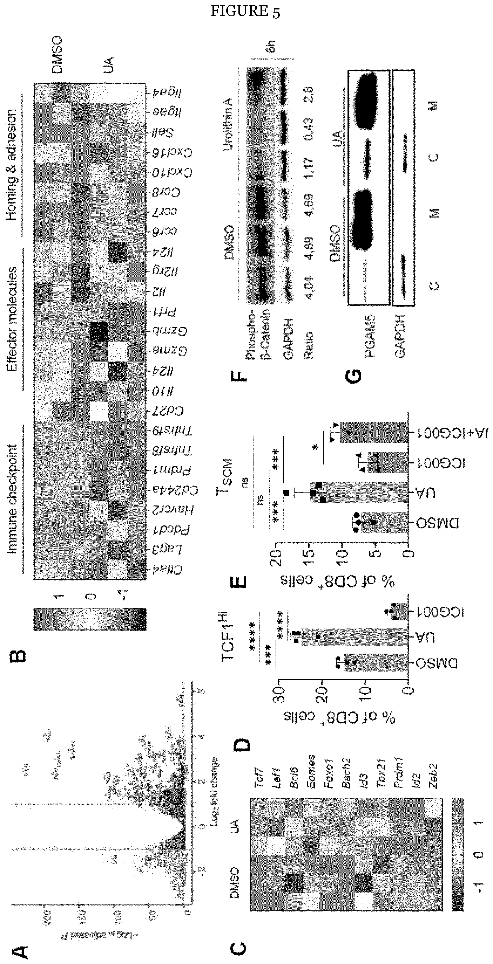

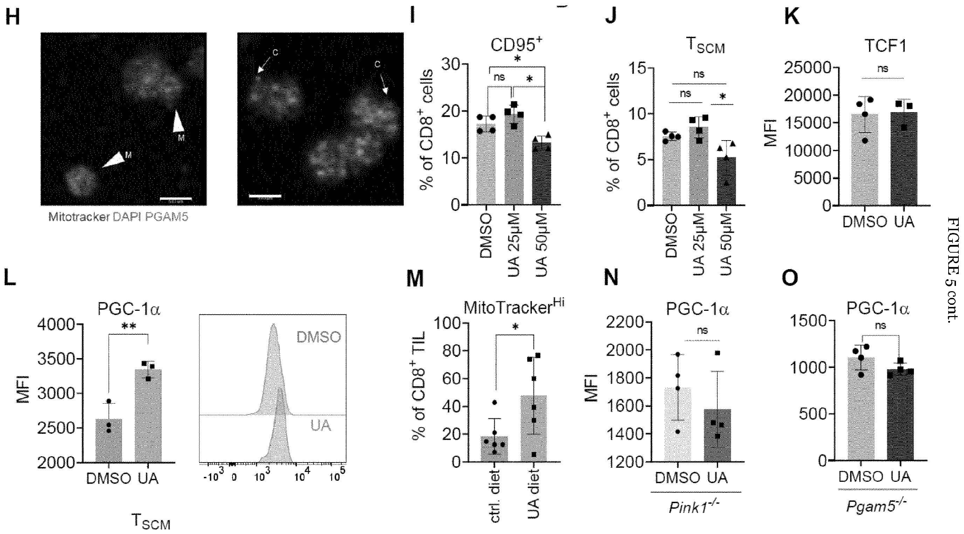

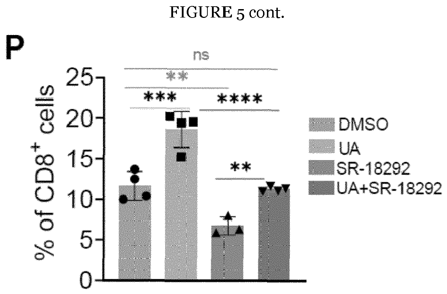

**Figure 5****: UA-induced T_{SCM} expansion is due to cytosolic release of PGAM5 that drives Wnt signalling (A)** Enhanced volcano plot of RNAseq of T cells stimulated for 48h with aCD3/aCD28 in the absence or presence of UA (50µM). A log2 fold change of 1 and a p-value of p<0.05 were considered significant (red, significantly upregulated in UA treatetd cells; green, significantly upregulated in DMSO treated cells). **(B)** Heatmap of differentially expressed genes associated with immune checkpoints, effector molecules and genes coding for leukocyte migration. **(C)** Heatmap of differentially expressed genes associated with T cell memory vs effector fate decisions. Data underwent z-score normalization for display (n=3 per group). **(D)** Frequency of TCF1^{Hi} expressing CD8⁺ T cells after 48h stimulation with aCD3/aCD28 in the presence of UA (50µM) or the TCF1 inhibitor ICG001 (10 µM) in comparison to DMSO control. Data are mean ± SD, n=₄, ^{∗∗∗}p<0.001; ^{∗∗∗∗}p<0.0001 by one-way ANOVA followed by Tukey's multiple comparison test. **(E)** Frequency of T_{SCM} after 48h stimulation with aCD3/aCD28 in the presence of UA (50µM) and the TCF1 inhibitor ICG001 (10 µM) in comparison to DMSO control. Data are mean ± SD, n=4: DMSO, UA, ICG001, n=3: UA+ICG001, ^{∗}p < 0.05 , p^{∗∗∗} <0,001; n.s. not significant by one-way ANOVA followed by Tukey's multiple comparison test. One of two independent experiments are shown. **(F)** Immunoblot analysis of phospho-P-catenin in T cells stimulated for 6h with aCD3/aCD28 in the absence or presence of UA (50µM). **(G)** Immunoblot analysis of fractionated T cells stimulated for 6h with aCD3/aCD28 in the absence or presence of UA (50µM); c= cytosolic, m = mitochondrial fraction. Experiment was repeated twice. **(H)** Immunofluorescence of PGAM5 (green) in T cells stimulated for 6h with aCD3/aCD28 in the absence or presence of UA (50µM). Cells were stained with MitoTracker Red to visualize mitochondria (m, mitochondrial localization; c, cytoplasmatic localization). Scale bar = 5µm. **(I-K)** Frequency of **(I)** T_{SCM}, **(J)** CD₉₅⁺, **(K)** TCF1 expressing *Pgam₅*^{*-*/*-*} CD8⁺ T cells after 48h stimulation with aCD3/aCD28 in the absence or presence of UA. Data are mean ± SD, n=4/group, ^{∗}p < 0.05, n.s. not significant by one-way ANOVA followed by Tukey's multiple comparison test. **(L)** PGC-1a expression in CD8⁺ T cells after 48h stimulation with aCD3/aCD28 in the absence or presence of UA (50µM). Data are mean ± SD, n=3, p^{∗∗}<0.01 by two-sided t-test. One of two independent experiments are shown. **(M)** Mitochondrial content of CD8⁺ TIL from APTK-induced tumors from *wt* mice fed UA or control diet as depicted in Fig. 1K. Data are mean ± SD, n=6/group, ^{∗}p < 0.05 by two-sided t-test. **(N, O)** PGC-1a expression in CD8⁺ T cells derived from *Pink1*^{*-*/*-*} KO mice (N) or *Pgam5*^{*-*/*-*} mice (O) after 48h stimulation with aCD3/aCD28 in the absence or presence of UA (50µM). Data are mean ± SD, n=4. **(P)** Frequency of T_{SCM} after 48h stimulation with aCD3/aCD28 in the presence of UA (50µM) or the PGC-1a inhibitor (PGC-1ai, 10 µM). Data are mean ± SD, n=₄, p^{∗∗}<0.01, ^{∗∗∗}p <0.001, ^{∗∗∗∗}p<0.0001 n.s. not significant by one-way ANOVA followed by Tukey's multiple comparison test.

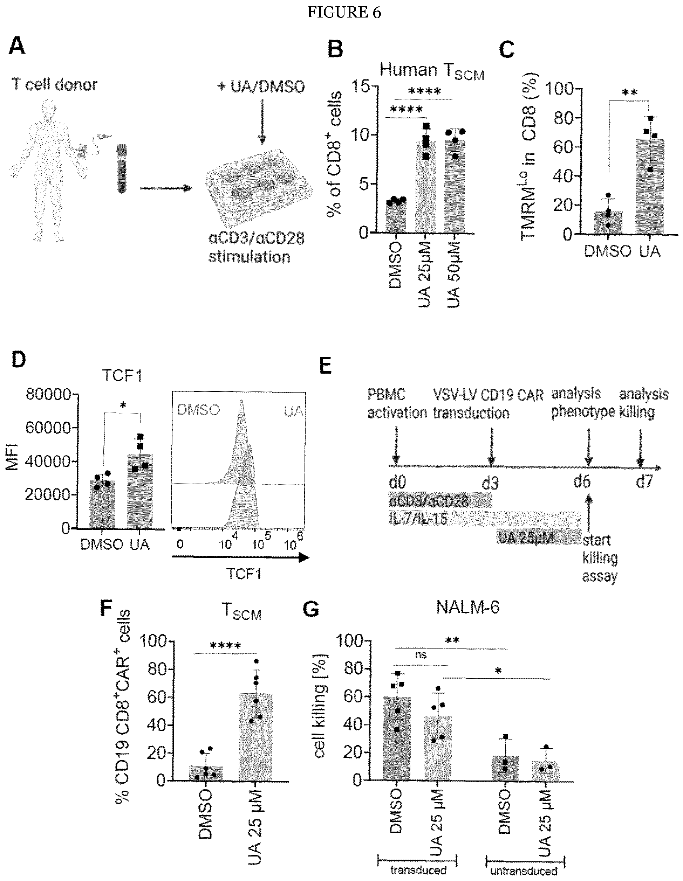

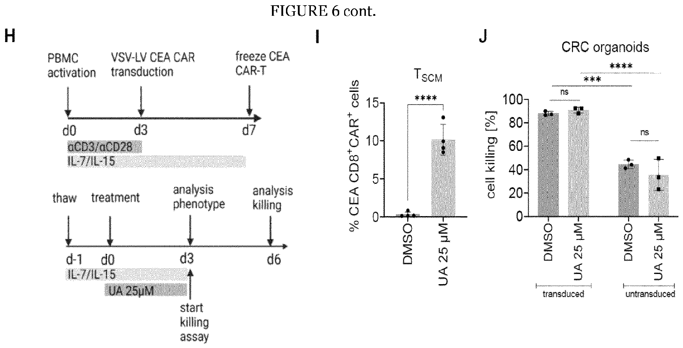

**Figure 6****: UA promotess human T_{SCM}, facilitating generation of potent CAR T_{SCM} (A)** Human PBMC were isolated from healthy donors, T cells were purified and stimulated *ex vivo* with aCD3/aCD28 in the presence of UA (50µM) or DMSO control. **(B)** Frequency of human T_{SCM} 48h after stimulation as shown in (A). Data are mean ± SD, p^{∗∗∗∗}0.0001 by one-way ANOVA followed by Tukey's multiple comparison test. **(C)** Quantification of human TMRM^{lo}CD8⁺ T cells 48h after stimulation in the presence of UA or DMSO control, Data are mean ± SD, p^{∗∗}0.01 by two-sided t-test. **(D)** TCF1 expression in human CD8⁺ T cells 48h after stimulation in the presence of UA or DMSO control, Data are mean ± SD, p^{∗}0.05 by two-sided t-test. In (B), representative data of one out of five donors with comparable outcome are shown. **(E)** Experimental layout of CD19-CAR T cell generation and expansion. PBMCS from healthy donors were expanded in the presence of IL-7/IL-15 for three days, followed by VSV-LV transduction. After three days of incubation, killing of Nalm-6 cells is assessed upon 24h co-culture. **(F)** Frequency of T_{SCM} within CD19-CAR⁺CD8⁺ cells after generation as depicted in (E). Data are mean ± SD, p^{∗∗∗∗}0.0001 by two-sided t-test. Data pooled from three independent experiments. **(G)** Killing potential of CD19-CAR-T cells. Percentage of dead NALM-6 cells upon 24h co-culture with untransduced or CAR-transduced T cells ±UA/DMSO is shown. Data are mean ± SD, n=5/3 (transduced/untransduced); p^{∗}0.05, p^{∗∗}<0.01, p^{∗∗∗∗}0.0001 by two-way ANOVA followed Sidak's multiple comparison test. **(H)** Experimental layout of CEA-CAR T cell experiments. Following CAR gene transduction (upper panel), CAR T cells were frozen for subsequent experiments after thawing (lower panel). **(I)** Frequency of T_{SCM} within CAR⁺CD8⁺ cells specific for CEA. Data are mean ± SD, n=₄, p^{∗∗∗∗}0.0001 by two-sided t-test. Data was pooled from two independent experiments. **(J)** Killing potential of CEA-specific CAR-T cells. Percentage of dead CEA-expressing human CRC organoids upon 72h co-culture with untransduced or CAR-transduced T cells ±UA/DMSO is shown. Data are mean ± SD, n = 3; ^{∗∗∗}p <0.001, p^{∗∗∗∗}0.0001, n.s. not significant by two-way ANOVA followed Sidak's multiple comparison test.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

### The examples show:

### Example 1: Urolithin A suppresses intestinal tumor growth in a T celldependent manner

To examine whether Urolithin A (UA)-dependent mitophagy mimicked the effect of *Stat3*^{DIEC} mice (Ziegler et al., 2018) and prevented intestinal tumor development in a T cell dependent manner, the inventors employed a model of azoxymethane (AOM)-induced tumorigenesis. FVB mice were injected with the procarcinogen AOM once a week for six weeks and kept for 18 weeks on an UA containing diet (2.28g/kg) or control diet (Fig. 1A). Oral UA administration led to a significant decrease in tumor incidence and tumor size (Fig. 1B-D). Expectedly, this was accompanied by an increased infiltration of CD3⁺ T cells into the colonic mucosa (Fig. 1E, F). To confirm UA-induced mitophagy and to explore whether UA could be employed therapeutically in more advanced colorectal cancer, the inventors took advantage of a recently developed tumor organoid system (Nicolas et al., 2022). The inventors treated APTK organoids (characterized by loss of *Apc, Trp53* and *Tgfbr2* as well as expression of oncogenic Kras^{G12D}) for 48 hours with increasing concentrations of UA (Fig. 1G) and observed a dose-dependent formation of lysosomes (Fig. 1H) and a concomitant loss of MitoTracker staining (Fig. 1I). This was paralleled by a marked upregulation of MHC-I (Fig. 1J) validating the inventor's previous observation linking mitophagy in IEC to MHC-1 upregulation (Ziegler et al, 2018). To examine the therapeutic potential of UA, the inventors subcutaneously (s.c.) transplanted APTK organoids into C57BL/6 mice (Fig. 1K). Tumor-bearing mice were subsequently subjected to the UA-supplemented diet, which resulted in significantly reduced tumor growth and enhanced CD8⁺ T cell infiltration (Fig. 1L-N). When the inventors transplanted APTK organoids s.c. into *Rag1*^{*-*/*-*} mice, the absence of mature T and B cells abrogated the protective effect of UA (Fig. 10). Similarly, depletion of CD8⁺ T cells led to exacerbated tumor growth in both control and UA-fed cohorts (Fig. 1P-Q), confirming that UA-induced tumor suppression was T cell dependent. Consequently, the inventors confirmed that UA sensitized APTK tumors to PD-1 blockade, while immune checkpoint inhibition alone did not affect APTK-tumor growth (Fig. 1R, S).

### Example 2: UA promotes T_{SCM} differentiation

Mitochondrial remodelling has been previously associated with alterations in T cell fate (Buck et al., 2016; Yu et al., 2020b). Given the observed dependency of the UA effect on CD8⁺ T cells, the inventors wondered whether UA may also directly affect T cell fate commitment. Purified T cells from FVB mice were stimulated with aCD3/aCD28 beads to induce T cell differentiation into effector T cell subsets in the presence or absence of UA for up to 72 hours (Fig. 2A). UA administration blocked differentiation into effector T cells (Fig. 2B, C) and resulted in a significant increase of naive CD44^{lo}CD62L^{hi} T cells (not shown). In addition, the inventors observed a reduction of central memory cells (T_{CM}), whereas the frequency of effector memory cells (T_{EM}) remained unaltered (not shown). In CD4⁺ cells, the inventors only observed changes at later timepoints, characterized by less naive-like CD4⁺ cells, but enhanced frequency of T_{EM} cells (not shown). Previously, a rare naive-like subset of T cells with enhanced sternness capabilities, termed T memory stem cells (T_{SCM}) has been identified (Gattinoni et al., 2009). T_{SCM} are marked by extreme longevity, their ability to self-renew, and potential for immune reconstitution (Gattinoni et al., 2017) which translates into potent anti-tumor immunity. Phenotypically, T_{SCM} represent a subset of minimally differentiated T cells, which share a CD44^{lo}CD62L^{hi} phenotype with naive T cells, but are phenotypically distinct by expressing high levels of *Sca1* (Gattinoni et al., 2009). Indeed, UA led to a significantly increased number of these CD44⁻CD62L⁺Sca1^{hi} T_{SCM} (Fig. 2D) that were characterized by a reduced mitochondrial membrane potential as well as increased CD95 expression (Fig. 2E, F). On the contrary, UA did not promote enhanced T_{SCM} formation in CD4⁺ cells (not shown). Furthermore, UA treatment led to dose-dependent T cell death *in vitro* (not shown) and restricted CD8⁺ T cell division (not shown). This was associated with reduced cyclin D1 expression (not shown), in line with previous observations that link T_{SCM} reprogramming to halted proliferation (Gattinoni et al., 2009; Verma et al., 2021). Moreover, the inventors observed that T_{SCM} were only formed in activated T cells (not shown). Excluding the possibility that UA reduces TCR-mediated activation of T cells, thus limiting effector formation by retaining a naive-like state, the inventors found that UA treated T cells show equal phosphorylation of Stat1 (Gamero and Larner, 2000) compared to vehicle controls (not shown).

Also *in vivo,* UA administration led to a marked increase of CD8⁺CD44^{lo}CD62L^{hi}Sca1^{hi} T cells in APTK-induced tumors (Fig. 2G), yet no change in CD4⁺ T_{SCM} or in an antigen-presenting or immune-suppressing TME represented by dendritic cells (DCs), TAMs, or MDSC subsets (not shown) This indicates that Urolithin A directly acts on T cells, and their reduced exhaustion state is not simply a by-product of an altered TME in this model. Indeed, fitting the observation of self-dividing memory subsets (Kratchmarov et al., 2018; Utzschneider et al., 2016), the inventors found increased expression of the *transcription factor 1* (TCF1) in tumor infiltrating CD8⁺ T cells (Fig. 2H). Additionally, the inventors observed a reduction of exhaustion markers such as PD-1, CTLA-4 and Tim3 in CD8⁺ T cells of UA-fed mice (Fig. 2I-K) which allowed stronger induction of TNFa and IFNg when tumor infiltrating CD8⁺ T cells were restimulated with PMA/ionomycin *ex vivo* (Fig. 2L, M). Collectively, this data indicates that UA treatment results in the formation of T_{SCM} in the TME, conferring superior CD8⁺-mediated antitumor immunity.

### Example 3: UA improves tumor therapy by adoptive T cell transfer

Adoptive cell transfer (ACT) represents the infusion of antigen-specific leukocytes with direct antitumor activity, yet identification, selection and expansion of lymphocyte subsets harboring optimal antitumor qualities remains one of the most crucial challenges (Rosenberg and Restifo, 2015). ACT benefits especially from minimally differentiated cells due to their improved survival and long-term potential to generate unexhausted effector cells (Luca Gattinoni et al; Mo et al., 2021; Roberto et al., 2015). In particular, CD8⁺ T cells restricted in a stem-like state have been associated with enhanced tumor suppressive properties upon adoptive cell transfer (Enrico Lugli et al; Verma et al., 2021). Having demonstrated that UA induces a T_{SCM} phenotype in murine T cells, the inventors next investigated whether UA could be exploited to improve adoptive immunotherapy. The inventors cultured T cells from OT-1 donor mice (Fig. 3A) for 48 hours in the presence of UA or vehicle control, followed by adoptive transfer into immundeficient *Rag1*^{*-*/*-*} mice. Prior to ACT, CD8⁺ T cells from OT-1 donors also displayed a T_{SCM} phenotype (not shown) with enhanced CD95 expression (not shown). Seven days following the transfer the number of engrafted CD8⁺ T cells was significantly higher in mice that had received Urolithin A-treated T cells (Fig. 3B) supporting an increased potential to expand. Moreover, when UA-conditioned OT-1 T cells were transplanted into mice bearing palpable ovalbuminoverexpressing APTK (APTK-OVA) tumors (Fig. 3C), this led to a more substantial tumor suppression in comparison to control OT-I T cells (Fig. 3D, E). Analysis of tumors revealed upon UA-T cell transfer a lower expression of CD44 and a higher number of TCF1^{Hi}CD8⁺ tumor infiltrating T cells (Fig. 3F-G) in tumors in line with maintenance of a UA-induced memory phenotype (Schumann et al., 2015; Zhou et al., 2010). CD62L expression remained unchanged (Fig. 3H). Moreover, consistent with an improved memory response, animals receiving UApretreated T cells contained less Tim3^{Hi}PD1^{Hi} terminally exhausted CD8⁺ T cells in the TME (Fig. 3I). Thus, UA augments immune-mediated antitumor memory upon adoptive cell transfer.

### Example 4: Urolithin A induces Pinki-dependent mitophagy in T cells

Next the inventors examined whether the shift towards CD44⁻CD62L⁺Sca1^{Hi} T_{SCM} cells was triggered by UA-induced mitophagy in T cells. The inventors confirmed a reduction of mitochondrial membrane potential within six hours after UA administration in CD8⁺ T cells (Fig. 4A-B). This was accompanied by enhanced lysosome formation (Fig. 4C) and after 24 hours followed by the loss of mitochondrial content in a dose-dependent manner (Fig 4D). The latter could be deteted in all T cell subsets analyzed (T_{SCM}, T_{CM} and T_{EFF}; Fig. 4E), thus suggesting induction of mitophagy.

UA activates mitophagy in myocytes and hippocampal neurons via a Pink1/Parkin mediated stress-response (D'Amico et al., 2021). Also in aCD3/aCD28 stimulated T cells, UA led to a significant upregulation of *Atg5, Atg7, p62, Lamp2* and *Pink1* gene expression (Fig. 4F). Moreover, UA stabilized Parkin protein expression (Fig. 4G). To functionally confirm the dependence of UA induced T_{SCM} formation on Pink1/Parkin mediated mitophagy, the inventors activated *Pink1*^{*-*/*-*} T cells in the presence or absence of UA. Loss of *Pink1* prevented the UA-induced lysosome formation as well as the decrease in mitochondrial content (Fig. 4H, I). *Pink1*^{-/-} CD8⁺ T cells failed to exhibit a T_{SCM} phenotype (Fig. 4J), and expressed less TCF-1 in CD44⁻CD62L⁺CD8⁺ T cells (Fig. 4K). Consequently, *Pink1* deletion abrogated the tumor suppressive effect of UA (Fig. 4L) while CD8⁺ TIL of *Pink1*^{*-*/*-*} mice did not exhibit differences in TCF-1 expression, exhaustion markers, or *ex vivo* cytokine release (Fig. 4M-Q) strongly supporting the notion that UA-induced Pink1-dependent mitophagy triggers T_{SCM} formation to enhance antitumor immunity.

### Example 5: UA induces T_{SCM} via cytosolic release of PGAM5 that drives Wnt signalling

To explore the downstream events linking mitophagy to T_{SCM} formation, the inventors performed RNA sequencing to globally assess differential gene expression in T cells exposed for 48 hours to either DMSO or UA in *vitro.* The inventors identified a total number of 1178 differentially expressed genes of which 765 were significantly upregulated and 413 downregulated (Fig. 5A). UA treatment reduced the expression of genes that code for immune checkpoints and effector molecules, while enhancing expression of *Cd27, Ccr7* and adhesion genes (Fig. 5B), a characteristic of stem-cell like CD8⁺ T cells (Enrico Lugli et al; Mo et al., 2021; Parisi et al., 2020; Reschke et al., 2021). Additionally, UA-treated cells revealed a special enrichment of genes involved in memory formation such as *Tcf7, Bach2* and *Bcl6* and reduced expression of effector fate associated genes *Prdm1* and *Id2* (Ichii et al., 2002; Roychoudhuri et al., 2016; Zhou et al., 2010) (Fig. 5C). In agreement with the increased number of TCF1^{Hi} T_{SCM} cells, upstream regulator analysis of RNAseq data revealed *Tcf7* as a possible regulator of UA-induced transcriptomic changes on T cells *in vitro* (not shown). Indeed, when the inventors applied ICG001 to pharmacologically block TCF-1, UA-induced T_{SCM} formation *in vitro* was abrogated (Fig. 5D-E).

Next the inventors examined whether the observed upregulation of TCF1 was a result of enhanced Wnt signalling. In line with this notion, the inventors observed an increased transcription of several Wnt target genes (Extended Data Fig. 4B). Moreover, UA led to a marked decrease of b-catenin phosphorylation already after 6 hours (Fig. 5F) indicating activation of Wnt-signaling prior to the transcriptional changes. Mitophagy releases the mitochondrial-bound protein phosphatase *phosphoglycerate mutase family member 5* (Pgam5) to the cytoplasm where it has been suggested to block axin-dependent b-catenin degradation thereby inducing mitochondrial biogenesis (Bernkopf et al., 2018; Yamaguchi et al., 2019). To explore whether Pgam5 may be involved in UA-induced Wnt activation, the inventors examined Pgam5 localization upon UA administration. Immunoblot analysis of sub-cellular fractionations revealed that Pgam5 was indeed rapidly released into the cytoplasma upon UA-mediated mitophagy (Fig. 5G), which could also be confirmed by immunofluorescence (Fig. 5H). More importantly, Pgam5 loss blocked the UA-dependent expansion of CD44⁻CD62L⁺Sca1^{H} T_{SCM} cells (Fig. 5I). UA treated Pgam^{-/-} CD8⁺ T cells further failed to upregulate TCF1 or the memory marker CD95 *in vitro* (Fig. 5J-K), despite the fact that UA still enhanced lysosome formation and reduced mitochondrial content (not shown) confirming that cytosolic Pgam5 contributed to UA-induced alterations of T cell function. In line with this notion, the inventors confirmed lack of cytoplasmic expression of Pgam5 in *Pink1*^{*-*/*-*} CD8⁺ T cells upon UA exposure (not shown).

Pgam5 dependent Wnt activation has been suggested to trigger compensatory mitochondrial biogenesis in response to mitophagy (Bernkopf et al., 2018). Peroxisome proliferator-activated receptor gamma coactivator 1-alpha (PGC-1α) is considered the master regulator of mitochondrial biogenesis (Finck and Kelly, 2006). Accordingly, the inventors detected a marked upregulation of PGC-1 after prolonged UA administration *in vitro* (Fig. 5L), which was accompanied by an increased mitochondrial content in TIL of APTK tumors (Fig. 5M), indicating mitochondrial biogenesis and in line with superior T cell fitness in the TME (Dumauthioz et al., 2020; Scharping et al., 2016). UA failed to enhance PGC-1a in *Pink1*^{*-*/*-*} or *Pgam5*^{*-*/*-*} CD8⁺ T cells (Fig. 5N, O), while chemical inhibition of PGC-1a blocked UA-dependent expansion of CD44⁻CD62L⁺Sca1^{H} T_{SCM} cells (Fig. 5P). Collectively, these results indicated that UA drives T_{SCM} formation via a Wnt-dependent upregulation of PGC-1a, promoted by the cystosolic release of Pgam5 in response to mitophagy.

### Example 6: UA promotess human T_{SCM}, facilitating generation of potent CAR T_{SCM}

Finally, the inventors aimed to determine whether UA causes expansion of T_{SCM} cells in human CD8⁺ T cells. The inventors isolated human CD3⁺ T cells from PBMCs of healthy donors and stimulated them with aCD3/aCD28 beads *in vitro* in the presence of UA (Fig. 6A). Indeed, UA increased the frequency of human T_{SCM} cells based on the expression of CD45RA⁺CCR7^{Hi}CD62L⁺CD95⁺CD8⁺ in five out of five individual donors (Fig. 6B). Like in murine T cells, after 48 hours UA-treated human CD8⁺ T cells displayed a decreased mitochondrial membrane potential (Fig. 6C) and intracellular staining confirmed increased TCF1 expression (Fig. 6D) validating that UA induces memory stem cell features in both murine and human T cells.

Infusion of T cells carrying an engineered chimeric antigen receptor (CAR), designed to specifically guide leukocytes to recognize and eliminate malignancies of interest, has shown impressive clinical results, but incomplete remissions and frequent relapses after successful therapy highlight the need to improve sustained antitumor response (June et al., 2018; Majzner and Mackall, 2019). Recent data suggests that CAR-T cells containing high fractions of T_{N}/_{SCM} show improved tumor killing and the exclusive ability to counteract leukemia re-challenge in hematopoietic stem/precursor cell-humanized mice (Arcangeli et al., 2022). To determine whether UA-triggered mitophagy also constitutes a feasible strategy to induce CAR-T_{SCM}, activated T cells were transduced with the CD19-CAR gene by a lentiviral vector (VSV-LV) in the presence or absence of UA. Three days post transduction, the amount of CAR-expressing T_{SCM} was determined (Fig. 6E). UA did not impair gene delivery into CD8⁺ cells (not shown), yet while CAR expressing T_{SCM} cells were markedly increased and comprised about 60% of CD8⁺ cells upon UA exposure (Fig. 6F), this did not have a negative impact on CD19 CAR-T cell mediated killing of NALM-6 leukemia cells (Fig. 6G). Markers of exhaustion were not affected by UA supplementation (not shown). Even when the inventors applied UA to previously frozen CAR-T cells specific for carcinoembryonic antigen (CEA; Fig. 6H), this strongly enhanced CAR T_{SCM} formation (Fig. 61) with comparable killing efficacy of CEA-expressing human CRC organoids (Fig. 6J). Thus, UA markedly enhances expansion of CAR-T_{SCM} cells.

### MATERIALS AND METHODS

### In vitro T cell isolation and stimulation.

Splenic murine T cells were isolated from mice aged 6-20 weeks by negative selection with EasySep mouse T cell isolation kit (Stem cell) according to the manufacturer's instructions. To monitor T cell proliferation, purified T cells were loaded with CellTrace Violet (C34557 ThermoFisher Scientific). T cell activation was initiated using αCD₃/CD28 activation beads (11456D; ThermoFisher Scientific) at a ratio of 25µl dynabeads per 0,5×10⁶ T-cells. Cells were activated with 25-50 µM Urolithin A (Tocris) or DMSO in T cell activation Medium (RPMI (ThermoFisher Scientific) 10% FBS (South America origin), 10mM Hepes (Sigma),1x Non-Essential Amino Acid, imM Sodium Pyruvate, 50µM β-Mercaptoethanol, 100 U/ml penicillin, 100 µg/ml streptomycin and 2mM Glutamax (ThermoFisher Scientific) for the indicated durations. In selected experiments, cells were stimulated in the presence of inhibitors, namely TCF1i (ICG-001; SelleckChem; 10µM) and PGC-iai (SR-18292, Sigma-Aldrich; 10µm,). For subsequent analysis, activating beads were magnetically removed prior antibody staining. For human T cells, peripheral blood mononuclear cells were purified with a Ficoll-Paque (GE healthcare) gradient. T cells were enriched with EasySep Human T Cell Isolation Kit (StemCell) and activated with dynabead Human T-Activator CD3/CD28 for T Cell Expansion and Activation Kit (11131D; ThermoFisher Scientific), similar to murine T cell stimulation.

When the subsequent activation and expansion of CAR T cells was studied, plates were coated with 1 µg/mL anti-human CD3 (aCD3; clone OKT3) and thawed PBMC were cultivated with 3 µg/mL anti-human CD28 (aCD28; 15E8, both Miltenyi Biotec) containing media for three days. (NutriT media (4Cell^{®} Nutri-T media (Sartorius) containing 0.5% penicillin/streptomycin) supplemented with IL-7 (25 U/ml) and IL-15 (U/ml) (all Miltenyi Biotec))

### Organoids.

APTK colorectal organoids which harbor mutations for *Apc, Trp53* and *Tgfbr2* as well as expression of oncogenic Kras^{G12D} were established and maintained as previously described (Nicolas et al., 2022; Varga et al., 2020). APTK organoids were seeded in 6 wells suspension plates: 6 droplets of 50 µl Matrigel (Corning) per well were used. To generate OVA-APTK, colon organoids were isolated from unchallenged OVA mice (The Jackson Laboratory) as previously described (Nicolas et al., 2022). Liposomes CRISPR/Cas9 technology- based transfection was used for targeted knockout of the following mutated colorectal cancer-related genes: *Apc* (gRNA: TATGGAACCTGTCTGCACACTGCAC), *Trp53* gRNA: GACACTCGGAGGGCTTCACT) and *Tgfbr2* (gRNA: GGCCGCTGCATATCGTCCTG) as previously described (Schwank and Clevers, 2016). Cas9 plasmid (Addgene; #41815) was used along gRNA plasmids generated from addgene #41819 plasmid by cloning the gRNA of interest instead of GFP targeting gRNA. Firstly, *Apc* knockout (A) organoids were functionally selected by the withdrawal of R-spondin from the culture medium. Secondly, *Apc Trp53* (AT) knockout organoids were selected for *Trp53* loss with *5* µM of MDM2 inhibitor (Nutlin-3) (Biomole, cat no. 10004372). Thirdly, *Apc Trp53 Tgfbr2* (APT) knockout organoids were selected with 5 µM recombinant TGFβ (R&D Systems; 7666-MB). Finally, Kras^{G12D} gain of function mutation was obtained by overexpressing the active murine version of *Kras* gene (cloned in house based on Addgene plasmid #111164, Map-S5) in APT organoids. *Apc, Trp53, Tgfbr2* and Kras^{G12D} (APTK) OVA organoids were selected by 2 µg/mL puromycin (Merck; 58-58-2) and withdrawal of EGF and noggin from the culture medium. In order to study the effect of UA on lysosome formation, mitochondria reduction as well as antigen presentation, organoids were treated with 25-50 µM UA for 24-48h. Organoids were subsequently stained with a MHC-I antibody (AF6-88.5; 116516; BioLegend), Mitotracker Deep Red (250nm; ThermoFisher) and Lysotracker Red DND-99 (75nM; ThermoFisher).

Human CRC organoids were established and cultured as previously described (Sato et al., 2011; van de Wetering et al., 2015). Organoids were cultured in medium composed of advanced DMEM/F12 supplemented with 20% R-spondin CM, 10% Noggin CM, 10 mM HEPES, 1× GlutaMax, 1× penicillin/streptomycin, 2% B27, 10 mM nicotinamide (Sigma-Aldrich), 12.5 mM N-acetylcystein (Sigma-Aldrich), 500 nM A83-01 (Tocris), 10 µM SB202190 (Biotrend), and 50 ng/ml human EGF (PeproTech). Unless stated otherwise, all media components were purchased from Life Technologies.

### Cell culture

Nalm-6 cells were cultivated in RPMI 1640 (Sigma, R0883-500 mL) medium supplemented with 10% fetal bovine serum (FBS; Sigma, F7524) and 2 mM glutamine (Sigma, G7513-100 ml). Their identity was confirmed by genetic phenotyping performed by the German cell culture collection (DSMZ).

### Colorectal cancer models.

Colorectal tumors were induced by 6 consecutive weekly injections of AOM on mice fed with food containing 2,28g/kg Urolithin A (UA diet) or with control food. The diet was introduced one-week prior the first AOM injection and maintained until the end of the experiment at ~20-24 weeks. The colons were collected for subsequent histological analysis as described below.

For subcutaneous colorectal cancers, APTK organoids were mechanistically disrupted, incubated with Accutase to generate a single cell suspension and reconstituted in PBS 20% Matrigel (Corning) for subcutaneous transplantation. Tumor sizes were measured at the indicated time after transplantation and tumor volume quantified as ½(width² × length). Urolithin A or control food regiment were initiated either one week before tumor challenge or 11 days after tumor development. Therapeutic diets were maintained throughout the experiment unless indicated otherwise. 200µg CD8-depleting antibodies (InvivoMab anti-mouse CD8; Hoelzel; BE0061) or isotype controls (InvivoMab rat IgG2b isotyp; Hoelzel; BE0090) were applied every three days, starting at five days after tumor initiation. To study combination efficacy with immunotherapy, mice were injected i.p. with 100µl nVivoMAb anti-mouse PDi(Hoelzel; BE0146) and InVivoMab mouse IgG2b isotype control (Hoelzel; BE0086) every 2 days, starting three days after tumor injection.

### Adoptive cell transfer

For adoptive cell transfer transfer, purified splenic T cell from OT1 mice were isolated and activated using aCD3/aCD28 stimulation beads for 48h. After magnetic bead removal, 1×10⁶ T of activated T cell were intravenously injected into *Rag1*^{*-*/*-*} recipients. To study survival of transferred T cells, stimulated OT-1 T cells were injected into mice without tumors. Subsequently, after 7 days, spleens were isolated and splenocytes were stained for the CD8⁺ subsets as described below. To understand antitumor immunity conferred by transferred OT-1 lymphocytes, cells were injected into mice carrying APTK-OVA tumors 10 days after subcutaneous organoid transplantation. Tumor growth was assessed as described above. Mice were kept under regular chow food for the T cell adoptive transfer experiment and sacrificed collectively when maximum tumor diameter extended 1,5 cm.

### Lentiviral (LV) production

Lentiviral vectors (LVs) were produced as described previously in detail for receptor targeted LVs (Weidner et al., 2021). In brief, HEK293T cells (ATCC CRL-11268) were transfected with a VSVG envelope (pMD2.G, Addgene: 12259), transfer and packaging plasmid in a ratio of 35:100:65 in presence of polyethylenimine (PEI; Sigma-Aldrich, 408727-100ML). The transfer vector plasmids encoded second generation CARs. The CEA-CAR encoding plasmid (Hombach et al., 2001) was modified by inserting the coding sequence for the reporter ΔLNGFR. The CD19-CAR contains a myc-tag for detection and was described (Pfeiffer et al., 2018). LVs were harvested from culture supernatants 48h after transfection, concentrated by a 24h centrifugation through a 20% sucrose cushion (Sigma, 84097) at 4,500 × g (4°C), and resuspended in Dulbecco's phosphate-buffered saline (DPBS, Mg2+ and Ca2+ free; Biowest, L0615-500). LVs were stored at -80°C and thawed only once for each experimental use.

### CAR gene delivery

For transduction, fully activated PBMC were seeded at 8×10⁴ cells/well in NutriT media supplemented with IL-7 and IL-15 in a 96-well plate. 0.5 µL VSV-LV was added to the cells, following spinfection via centrifugation for 90 min at 850xg and 32°C. After spinfection, 100 µL of fresh media was added. Optionally, medium was replaced with fresh medium containing 25 µM UA or DMSO 5 h post incubation with VSV-LV. Subsequently, cells were further cultivated at 37°C. Three days post transduction, CD19 CAR expression and the amount of T_{SCM} were determined. For CEA CAR T cells, T cells were frozen upon transduction and thawed for UA exposure experiments.

### CAR T cell killing assay

The cytotoxic activity of CD19-CAR T cells generated in presence or absence of UA was determined by CD19-positive Nalm-6 cell killing. Three days post transduction, CAR expression was determined and normalized to equal CAR T cell levels. Cells were washed twice and 0.5×10⁴ CAR T cells were seeded into NutriT medium without cytokines, UA or DMSO. Nalm-6 cells were labeled using CellTrace^{™} Violet (CTV; Thermo Fisher Scientific, C34557) following manufacturer's instructions and 1×10⁴ labeled Nalm-6 cells were added to the CAR T cells (0.5:1 effector to target ratio). The amount of dead target cells was determined via flow cytometry 24 h after start of killing.

In human CRC organoids, CEA-expression was confirmed by flow cytometry □CEACAM5-APC (487609; FAB41281A; R&D Systems) Before co-culture, CEA- CAR T cells were cultivated in presence or absence of UA for three days. The cytotoxicity assay was performed as described (Schnalzger et al., 2019), using 96 well plates coated with 15 µl Cultrex Basement Membrane Extract, Type 2 per well and organoids were seeded at a ratio of 1:8. Co-culture was performed for three days with 0.5×10⁴ CAR T cells per well resuspended in organoid medium (as described above) without IL7/IL15. 100 µl luciferase assay reagent composed of 10% One-Glo EX Reagent (Promega) in lysis buffer (50 mM NaCl, 50 mM Tris-HCl pH 7.4, 1% Triton X-100) was added to each well and incubated at room temperature for 1h protected from light. 100 µl of each sample was transferred to an opaque 96-well microtiter plate and measured using a SpectraMax iD3 microplate reader (Molecular Devices).

For freezing of CAR-T cells, pelleted cells were resuspended in 500µl of NutriT medium supplemented with 20% FCS. 500 µl of NutriT medium supplemented with 20% FCS and 20% DMSO was added dropwise. For thawing, a vial of CAR T cells heated for 1 min in a 37°C water bath. Thawed cells were washed twice with NutriT medium, centrifuged (5min, 330 g) and resuspended in NutriT medium supplemented with IL-7 (25 U/ml) and IL-15 (U/ml).

### Tumor collection and sample preparation

Tumors were minced and digested with Tumor & Tissue Dissociation Reagent (BD Horizon) for 30 min. The remaining organ pieces were filtered through a 70 µm cell strainer. Cells were then washed with PBS 1% BSA and 2mM EDTA. Immune cells were enriched on a 30%/40%/75% Percoll gradient after a 1700 rpm 17 min centrifugation. Then, tumor immune filtrates or activated T cells were stained with Fixable Viability Dye-eF780 (65-0865-14; eBioscience), αCD45-FITC (30-F11; 11-0451-85; eBioscience), αCD11b-BV650 (M1/70; 56340; BD) α-CD11c-BV785 (HL3; 563735; BD), αLy6G-BV605 (53-6.7; 563005; BD), αLy6C-AF700 (HK1.4; 128024; Biolegend), αF4/80-PE (BM8; 12-4801-82, eBioscience), αPD-L1-PECy7 (MIH5; 25-5982-80, eBioscience); or with Fixable Viability Dye-eF780 (ebioscience), αCD45-BV786 (30-F11; 564225; BD), αCD4-BUV496 (GK1.5; 564667; BD), αCD8-BV605 (100744; Biolegend), αPD1-APC (J43; 17-9985-82; eBioscience), αTIM-3-PECy7 (RMT3-23; 119715; Biolegend), αCTLA4-PE (UC10-4B9; 106305; Biolegend) and αLag3-BV421 (C9B7W; 125221, Biolegend); or with Fixable Viability Dye-eF780 (eBioscience), αCD4-FITC (H129.19; 553650; BD), αCD8-APC (53-6.7; 17-0081-83; eBioscience), αCD44-AF400 (IM7; 103026; Biolegend), αCD62L-BV785 (MEL-14; 104440; Biolegend), αSea-1-PerCP-Cy5.5 (D7; 45-5981-82; eBioscience), αCD95-PE (Jo2; 554258; BD); or with Fixable Viability Dye-eF780 (eBioscience), αCD8-BV650 (53-6.7; 563234; BD), αCD4-BUV496 (GK1.5; 564667; BD), αCD127-APC (A7R34; 17-1271-82; eBioscience), αCD95-FITC (SA367H8;152605; Biolegend), αCD44-AF400 (IM7; 103026; Biolegend), αCD62L-BV785 (MEL-14; 104440; Biolegend), αSca-1-PerCP-Cy5.5 (D7; 45-5981-82; eBioscience) followed by intracellular staining ofαTCF1-PE (S33-966; 564217; BD), or PGC-1α (sc-518025; D-5; santa cruz), or αCyclinD1 (MA₅-28534, DCS-6; Invitrogen) with transcription factor buffer set kit (BD Biosciences). To study cytokine expression, tumor immune infiltrates were stimulated *ex vivo* with 20ng/ml PMA and 1µg/ml Ionomycin (Sigma) for 3h in T cell activation medium with Brefeldin A (Biolegend). For T cell *in vitro* activation experiments over several days, Brefeldin A was added to the medium for the last 3 hours. Cells were then stained with Fixable Viability Dye-eF780 (eBioscience), αCD8-PeCy7 (53.6-7; 100722; Biolegend), αCD4-AF700 (GK1.5; 100429; Biolegend), and intracellular staining for cytokine assessment was performed with BD Cytofix/Cytoperm (BD) with 20 min fixation and overnight incubation with αGranzyme B-FITC (GB11; 515403; Biolegend), αIFNγ-PerCP-Cy5.5 (XMG1.2; 45-7311-82; eBioscience) and αTNFa-BV711 (MP6-XT22; 563944; BD).

For flow cytometric analysis of mitochondria and lysosomes 500µl of Mitotracker Red (200nM; ThermoFisher) or Lysotracker Red DND-99 (75nM; ThermoFisher) were applied followed by surface staining. To study changes in mitochondrial membrane potential, Mitoprobe TMRM kit (ThermoFisher) was used according to the manufacturer's instructions. Cell proliferation was assessed by CellTrace Violet (Invitrogen, C34557) at a concentration of 5µM according to manufacturer's instructions. For human samples, cells were stained as described above with Fixable Viability Dye-eF780, αCD95-BV421 (DX2; 562616), αCD62L-PE (DREG-56; 555544), αCCR7-AF700 (150503; 561143), αCD45RO-APC (UCHL; 559865), αCD45RA-FITC (HI100; 555488), αCD8-PE-CF594 (RPA-T8; 562282) and αCD4-BV786 (OKT₄; 566806) from BD.

For experiments studying activation, expansion and exhaustion of CAR T cells, cells were stained with αCD45RA-VioBlue (T6D11; 130-113-922; Miltenyi), αmycCAR-FITC (SH1-26e7.1.3; 130-116-653; Miltenyi), αCD8- PerCP-Cy5.5 (RPA-T8; 560662; BD), CD62L-PE-Vi0770 (145/15; 130-113-621; Miltenyi), CD45RO-APC (UCHL1, 130-113-546; Miltenyi), Fixable Viability Dye-eF780; or mycCAR-FITC (SH1-26e7.1.3; 130-116-653; Miltenyi), αCD8- PerCP-Cy5.5 (RPA-T8; 560662; BD), αPD1- PE-Vi0770 (PD1.3.1.3; 130-117-810; Miltenyi), αTim3-APC (REA635; 130-119-781; Miltenyi) and Fixable Viability Dye-eF780.Finally, samples were fixed with 1% PFA until data acquisition.

### Flow cytometry data acquisition

Cell doublets and cells debris were excluded from the analysis by gating FSC-A vs FSC-H followed by SSC-A vs SSC-H dot plots and FSC-A vs SSC-A dot plots, respectively. eFluor 780 highly positive cells, i.e, dead cells, were gated out of the analysis as well as CD45 negative cells in the case of tumor samples. Data was acquired on Canto II (BD), Fortessa (BD) and Aurora (Cytek) cytometers and analyzed with FlowJo with the subsequent gating strategy described in the supplementary dataset.

### Quantitative PCR with reverse transcription analysis

*In vitro* activated T cells following the indicated treatments were collected followed by RNA isolation using the RNeasy kit (Qiagen). RNA quality and concentration were determined by Nanodrop spectrophotometer. Complementary DNA was generated using SuperScript II reverse transcriptase (ThermoFisher). A total reaction volume of 13 µl RNA was incubated with oligoDT and DNTP Mix (1 µl each) for 5 min at 65°C. The samples were allowed to cool down for 5 min on ice, and to each 4 µl of 5× First strand buffer, 1 µl DTT, 1 µl RNAseOUT^{™} and 1 µl Superscript II reverse transcriptase were added. Samples were incubated for 1 h at 42°C. Synthesized cDNA was 1:5 diluted in nuclease free water before being processed into gene expression analysis with the 96 wells StepOne real time PCR Machine (Applied biosystems) using 2× FastStart Universal SYBR Green Master Mix (Roche). CT values were normalized to the gene indicated in the respective figures. The following primers were used :
*Atg 5* (For: GGAGAGAAGAGGAGCCAGGT, Rev: GCTGGGGGACAATGCTAATA),
*Atg₇* (For: GCCTAACACAGATGCTGCAA, Rev: TGCTCTTAAACCGAGGCTGT),
*Atg12*(For: TAAACTGGTGGCCTCGGAAC, Rev: ATCCCCATGCCTGGGATTTG),
*Pik3c3* (For: GTGAAGTACCCTGACCTGCC, Rev: AGTCATGCATTCCTTGGCGA),
*p62* (For: GCTGAAGGAAGCTGCCCTAT, Rev: TTGGTCTGTAGGAGCCTGGT),
*Bnip₃* (For: ATGCACAGCATGAGTCGGG, Rev:CTGGTATGCATCTCAACATCAAACA),
*Lamp2* (TGTGCAACAAAGAGCAGGTG, Rev: TCCAGTATGATGGCGCTTGAG),
*Pinki* (For: AAGCACCAGAAATTGCGACG, Rev: ACGAGATGGGAGTGCTGGTA),
*TCF7* (For: CAATCTGCTCATGCCCTACC, Rev: CTTGCTTCTGCGTGATGTCC),
*Rplpo* (For: TTCATTGTGGGAGCAGAC, Rev: CAGCAGTTTCTCCAGAGC),
*Lef1* (For: GCAGCTATCAACCAGATCC, Rev GATGTAGGCAGCTGTCATTC),
*Axin2* (For: CAGAGGTGGTACCTTGCCAAA, Rev GCCGACAGTGCAAGACCCLIST),
*cmyc* (For: AACTACGCAGCGCCTCCC, Rev: ATTTTCGGTTGTTGCTGATCTGT).

### RNA seq

For RNA isolation of *in vitro* stimulated T cells cells, RNeasy mini kit (Qiagen) was used. 200ng of total RNA was used to prepare the library for RNA sequencing. Coexpression patterns of genes associated with effector and memory cell fate, immune checkpoints, effector molecules, and adhesion/homing were chosen based on the literature as depicted in the main text. Data underwent z-score normalization for display as described previously (Xiong et al., 2020). Upstream regulator analysis was performed using Ingenuity pathway analysis software, using a cut-off log2fold change of 1 and a p value of >0,05.

### Imaging

Activated T cells were incubated on Poly-L-lysine coated coverslips for 10 min at 37°C and then fixed with 4% PFA for 10min, followed by permeabilization with 0,25% Triton X-100 for 10 mins. Staining for Pgam5 was performed as previously described (Yamaguchi et al., 2019), using rabbit Anti-Pgams antibody (ab126534, Abcam). Immune complexes were detected with 1:200-diluted anti-rabbit IgG Alexa Fluor 488 (Thermo Fisher). Nuclei were counterstained with a DAPI-containing liquid m liquid mountant (ProLong^{™} Gold Antifade Mountant, Thermo Fisher). Mitochondria were stained with 200nM Mitotracker Red for 45min Image were acquired by confocal microscopy.

### Histology.

For histological analysis of colon tissue, colons were were paraffin embedded using the "swiss roll" technique and serially sectioned at 200 µm. After deparaffinization and rehydration of the tissue, haematoxylin and eosin (H&E) staining and α-CD3 (IS503: DAKO) was performed using a Leica Bond Max following standard immunochemistry staining protocols. Stained sections were scanned using Aperio CS2 (Leica). AOM tumor lesion size and incidence were quantified using Aperio ImageScope software.

### Protein analysis

5×10⁶ isolated T cells were stimulated on cell-culture plates coated with 1:200 α-CD₃ (145-2C11; 100339; BioLegend) and α-CD28 (37.51; 102115, Biolegend) for the indicated time points. After centrifugation for 5mins at 1500rpm, cell pellets were resuspended in complete protein lysis buffer (50 mM Tris pH 7.5, 250 mM sodium chloride,30 mM EDTA, 25 mM sodium pyrophosphate, 1% Triton-X 100, 0.5% NP-40, 10% glycerol and 1 mM DTT) supplemented with protease and phosphatase inhibitors (Roche). When subcellular location of Pgam5 was studied, a cell fractionation kit (Abcam, Ab109719) was used according to the manufacturer's instructions. Fractionation and whole-cell lysates were snap frozen and stored at -80°C until further analysis. Collected lysates were mixed with 10% β-mercaptoethanol Laemmli buffer and denatured for 10 min at 70°C. Equal amounts of protein as assessed by Bradford measurement (Pierce^{™} Coomassie Plus (Bradford) Assay Reagent, ThermoFisher) were subjected to 10-15% SDS-PAGE, then transferred to 0.45µM PVDF membranes (MeckMillipore). Membranes were blocked for 30 mins in PBS-T/5% milk or PBST-T/5%BSA prior to antibody incubation overnight with the following antibodies: β-Actin (Sigma; #A4700), Gapdh (Santacruz; sc32233), Parkin (Santacruz; sc32283), Phospho-β-Catenin (CellSignaling; 9561), TOM20 (CellSignaling; D84TN;42406S); pStati (Cellsignaling; 9167) and PGAM5 (Abcam; ab126534). Afterwards, membranes were incubated for one hour with their respective HRP-conjugated secondary antibodies (Santacruz: sc2314 and sc2313), followed by development using SuperSignal West Pico Chemoluminescence Substrate (ThermoFischer Scientific). 2D densitometry was perfomed using ImageJ Software (National Institutes of Health, Bethesda, Maryland).

### Quantification and statistical analysis

All statistical analyses were performed using GraphPad Prism 8. Number of samples and experiments, as well as statistical test used are reported in each figure legend. Sample size was determined by prior experience. Data shown as ±SEM or ±SD according to figure legend. Statistical significance was assessed by the test depicted in the respective figure legend (* p<0.05, ^{∗∗} p<0.01, ^{∗∗∗} p<0.001 and ^{∗∗∗∗} p<0.0001)

### REFERENCES

The references are:
Abou-El-Enein, M., Elsallab, M., Feldman, S.A., Fesnak, A.D., Heslop, H.E., Marks, P., Till, B.G., Bauer, G., Savoldo, B., 2021. Scalable Manufacturing of CAR T cells for Cancer Immunotherapy. Blood cancer discovery 2, 408-422. https://doi.org/10.1158/2643-3230.BCD-21-0084.
Andreux, P.A., Blanco-Bose, W., Ryu, D., Burdet, F., Ibberson, M., Aebischer, P., Auwerx, J., Singh, A., Rinsch, C., 2019. The mitophagy activator urolithin A is safe and induces a molecular signature of improved mitochondrial and cellular health in humans. Nature metabolism, 595-603. https://doi.org/10.1038/s42255-019-0073-4.
Arcangeli, S., Bove, C., Mezzanotte, C., Camisa, B., Falcone, L., Manfredi, F., Bezzecchi, E., El Khoury, R., Norata, R., Sanvito, F., Ponzoni, M., Greco, B., Moresco, M.A., Carrabba, M.G., Ciceri, F., Bonini, C., Bondanza, A., Casucci, M., 2022. CAR T-cell manufacturing from naive/stem memory T-lymphocytes enhances antitumor responses while curtailing cytokine release syndrome. The Journal of clinical investigation. https://doi.org/10.1172/JCI150807.
Bernkopf, D.B., Jalal, K., Brückner, M., Knaup, K.X., Gentzel, M., Schambony, A., Behrens, J., 2018. Pgam5 released from damaged mitochondria induces mitochondrial biogenesis via Wnt signaling. The Journal of cell biology 217,1383-1394. https://doi.org/10.1083/jcb.201708191.
Boakye, Y.D., Groyer, L., Heiss, E.H., 2018. An increased autophagic flux contributes to the antiinflammatory potential of urolithin A in macrophages. Biochimica et biophysica acta. General subjects 1862, 61-70. https://doi.org/10.1016/j.bbagen.2017.10.006.
Buck, M.D., O'Sullivan, D., Klein Geltink, R.I., Curtis, J.D., Chang, C.-H., Sanin, D.E., Qiu, J., Kretz, O., Braas, D., van der Windt, G.J.W., Chen, Q., Huang, S.C.-C., O'Neill, C.M., Edelson, B.T., Pearce, E.J., Sesaki, H., Huber, T.B., Rambold, A.S., Pearce, E.L., 2016. Mitochondrial Dynamics Controls T Cell Fate through Metabolic Programming. Cell 166, 63-76. https://doi.org/10.1016/j.cell.2016.05.035.
Chen, D.S., Mellman, I., 2017. Elements of cancer immunity and the cancer-immune set point. Nature 541, 321-330. https://doi.org/10.1038/nature21349.
Cortés-Martín, A., García-Villalba, R., González-Sarrías, A., Romo-Vaquero, M., Loria-Kohen, V., Ramírez-de-Molina, A., Tomás-Barberán, F.A., Selma, M.V., Espin, J.C., 2018. The gut microbiota urolithin metabotypes revisited: the human metabolism of ellagic acid is mainly determined by aging. Food & function 9, 4100-4106. https://doi.org/10.1039/C8FO00956B.
D'Amico, D., Andreux, P.A., Valdés, P., Singh, A., Rinsch, C., Auwerx, J., 2021. Impact of the Natural Compound Urolithin A on Health, Disease, and Aging. Trends in molecular medicine 27, 687-699. https://doi.org/10.1016/j.molmed.2021.04.009.
Dumauthioz, N., Tschumi, B., Wenes, M., Marti, B., Wang, H., Franco, F., Li, W., Lopez-Mejia, I.C., Fajas, L., Ho, P.-C., Donda, A., Romero, P., Zhang, L., 2020. Enforced PGC-1α expression promotes CD8 T cell fitness, memory formation and antitumor immunity. Cellular & molecular immunology. https://doi.org/10.1038/s41423-020-0365-3.
Enrico Lugli, Yun Ji, Zoltan Pos, Chrystal M Paulos, M&aacute, ire F Quigley, Jorge R Almeida, Emma Gostick, Zhiya Yu, Carmine Carpenito, Ena Wang, Daniel C Douek, David A Price, Carl H June, Francesco M Marincola, Mario Roederer, Luca Gattinoni, Nicholas P Restifo. A human memory T cell subset with stem cell–like properties.
Espin, J.C., Larrosa, M., García-Conesa, M.T., Tomás-Barberán, F., 2013. Biological significance of urolithins, the gut microbial ellagic Acid-derived metabolites: the evidence so far. Evidence-based complementary and alternative medicine : eCAM 2013, 270418. https://doi.org/10.1155/2013/270418.
Finck, B.N., Kelly, D.P., 2006. PGC-1 coactivators: inducible regulators of energy metabolism in health and disease. The Journal of clinical investigation 116, 615-622. https://doi.org/10.1172/JCI27794.
Gamero, A.M., Larner, A.C., 2000. Signaling via the T cell antigen receptor induces phosphorylation of Stat1 on serine 727. The Journal of biological chemistry 275, 16574-16578. https://doi.org/10.1074/jbc.M910149199.
Gattinoni, L., Speiser, D.E., Lichterfeld, M., Bonini, C., 2017. T memory stem cells in health and disease. Nature medicine 23,18-27. https://doi.org/10-1038/nm.4241.
Gattinoni, L., Zhong, X.-S., Palmer, D.C., Ji, Y., Hinrichs, C.S., Yu, Z., Wrzesinski, C., Boni, A., Cassard, L., Garvin, L.M., Paulos, C.M., Muranski, P., Restifo, N.P., 2009. Wnt signaling arrests effector T cell differentiation and generates CD8+ memory stem cells. Nature medicine 15, 808-813. https://doi.org/10.1038/nm.1982.
Ghosh, S., Haribabu, B., Jala, V.R., 2021. Microbial metabolite Urolithin A induces expansion of Regulatory T Cells in aryl hydrocarbon receptor (AhR)-dependent manner. The Journal of Immunology 206, 113.03.
Hombach, A., Wieczarkowiecz, A., Marquardt, T., Heuser, C., Usai, L., Pohl, C., Seliger, B., Abken, H., 2001. Tumor-specific T cell activation by recombinant immunoreceptors: CD3 zeta signaling and CD28 costimulation are simultaneously required for efficient IL-2 secretion and can be integrated into one combined CD28/CD3 zeta signaling receptor molecule. Journal of immunology (Baltimore, Md. : 1950) 167, 6123-6131. https://doi.org/10.4049/jimmunol.167.11.6123.
Ichii, H., Sakamoto, A., Hatano, M., Okada, S., Toyama, H., Taki, S., Arima, M., Kuroda, Y., Tokuhisa, T., 2002. Role for Bcl-6 in the generation and maintenance of memory CD8+ T cells. Nature immunology 3, 558-563. https://doi.org/10.1038/ni8O2.
Jaganathan, S.K., Vellayappan, M.V., Narasimhan, G., Supriyanto, E., 2014. Role of pomegranate and citrus fruit juices in colon cancer prevention. World journal of gastroenterology 20, 4618-4625. https://doi.org/10.3748/wjg.v20.i16.4618.
June, C.H., O'Connor, R.S., Kawalekar, O.U., Ghassemi, S., Milone, M.C., 2018. CAR T cell immunotherapy for human cancer. Science (New York, N.Y.) 359, 1361-1365. https://doi.org/10.1126/science.aar6711.
Kratchmarov, R., Magun, A.M., Reiner, S.L., 2018. TCF1 expression marks self-renewing human CD8+ T cells. Blood advances 2, 1685-1690. https://doi.org/10.1182/bloodadvances.2018016279.
Le, D.T., Uram, J.N., Wang, H., Bartlett, B.R., Kemberling, H., Eyring, A.D., Skora, A.D., Luber, B.S., Azad, N.S., Laheru, D., Biedrzycki, B., Donehower, R.C., Zaheer, A., Fisher, G.A., Crocenzi, T.S., Lee, J.J., Duffy, S.M., Goldberg, R.M., La Chapelle, A. de, Koshiji, M., Bhaijee, F., Huebner, T., Hruban, R.H., Wood, L.D., Cuka, N., Pardoll, D.M., Papadopoulos, N., Kinzler, K.W., Zhou, S., Cornish, T.C., Taube, J.M., Anders, R.A., Eshleman, J.R., Vogelstein, B., Diaz, L.A., 2015. PD-1 Blockade in Tumors with Mismatch-Repair Deficiency. The New England journal of medicine 372, 2509-2520. https://doi.org/10.1056/NEJM0a1500596.
Luan, P., D'Amico, D., Andreux, P.A., Laurila, P.-P., Wohlwend, M., Li, H., Imamura de Lima, T., Place, N., Rinsch, C., Zanou, N., Auwerx, J., 2021. Urolithin A improves muscle function by inducing mitophagy in muscular dystrophy. Science translational medicine 13. https://doi.org/10.1126/scitranslmed.abbo319.
Luca Gattinoni, Christopher A. Klebanoff, Nicholas P. Restifo. Paths to sternness: building the ultimate antitumour T cell. https://doi.org/10-1038/nrc3322.
Majzner, R.G., Mackall, C.L., 2019. Clinical lessons learned from the first leg of the CAR T cell journey. Nature medicine 25,1341-1355. https://doi.org/10.1038/s41591-019-0564-6.
Mo, F., Yu, Z., Li, P., Oh, J., Spolski, R., Zhao, L., Glassman, C.R., Yamamoto, T.N., Chen, Y., Golebiowski, F.M., Hermans, D., Majri-Morrison, S., Picton, L.K., Liao, W., Ren, M., Zhuang, X., Mitra, S., Lin, J.-X., Gattinoni, L., Powell, J.D., Restifo, N.P., Garcia, K.C., Leonard, W.J., 2021. An engineered IL-2 partial agonist promotes CD8+ T cell sternness. Nature 597, 544-548. https://doi.org/10.1038/s41586-021-03861-0.
Nicolas, A.M., Pesic, M., Engel, E., Ziegler, P.K., Diefenhardt, M., Kennel, K.B., Buettner, F., Conche, C., Petrocelli, V., Elwakeel, E., Weigert, A., Zinoveva, A., Fleischmann, M., Häupl, B., Karakütük, C., Bohnenberger, H., Mosa, M.H., Kaderali, L., Gaedcke, J., Ghadimi, M., Rödel, F., Arkan, M.C., Oellerich, T., Rödel, C., Fokas, E., Greten, F.R., 2022. Inflammatory fibroblasts mediate resistance to neoadjuvant therapy in rectal cancer. Cancer cell 40, 168-184.e13. https://doi.org/10.1016/j.ccell.2022.01.004.
Pages, F., Mlecnik, B., Marliot, F., Bindea, G., Ou, F.-S., Bifulco, C., Lugli, A., Zlobec, I., Rau, T.T., Berger, M.D., Nagtegaal, I.D., Vink-Börger, E., Hartmann, A., Geppert, C., Kolwelter, J., Merkel, S., Grützmann, R., van den Eynde, M., Jouret-Mourin, A., Kartheuser, A., Léonard, D., Remue, C., Wang, J.Y., Bavi, P., Roehrl, M.H.A., Ohashi, P.S., Nguyen, L.T., Han, S., MacGregor, H.L., Hafezi-Bakhtiari, S., Wouters, B.G., Masucci, G.V., Andersson, E.K., Zavadova, E., Vocka, M., Spacek, J., Petruzelka, L., Konopasek, B., Dundr, P., Skalova, H., Nemejcova, K., Botti, G., Tatangelo, F., Delrio, P., Ciliberto, G., Maio, M., Laghi, L., Grizzi, F., Fredriksen, T., Buttard, B., Angelova, M., Vasaturo, A., Maby, P., Church, S.E., Angell, H.K., Lafontaine, L., Bruni, D., El Sissy, C., Haicheur, N., Kirilovsky, A., Berger, A., Lagorce, C., Meyers, J.P., Paustian, C., Feng, Z., Ballesteros-Merino, C., Dijkstra, J., van de Water, C., van Lent-van Vliet, S., Knijn, N., Musina, A.-M., Scripcariu, D.-V., Popivanova, B., Xu, M., Fujita, T., Hazama, S., Suzuki, N., Nagano, H., Okuno, K., Torigoe, T., Sato, N., Furuhata, T., Takemasa, I., Itoh, K., Patel, P.S., Vora, H.H., Shah, B., Patel, J.B., Rajvik, K.N., Pandya, S.J., Shukla, S.N., Wang, Y., Zhang, G., Kawakami, Y., Marincola, F.M., Ascierto, P.A., Sargent, D.J., Fox, B.A., Galon, J., 2018. International validation of the consensus Immunoscore for the classification of colon cancer: a prognostic and accuracy study. The Lancet 391, 2128-2139. https://doi.org/10.1016/S0140-6736(18)30789-X.
Parisi, G., Saco, J.D., Salazar, F.B., Tsoi, J., Krystofinski, P., Puig-Saus, C., Zhang, R., Zhou, J., Cheung-Lau, G.C., Garcia, A.J., Grasso, C.S., Tavaré, R., Hu-Lieskovan, S., Mackay, S., Zalevsky, J., Bernatchez, C., Diab, A., Wu, A.M., Comin-Anduix, B., Charych, D., Ribas, A., 2020. Persistence of adoptively transferred T cells with a kinetically engineered IL-2 receptor agonist. Nature communications 11, 660. https://doi.org/10.1038/s41467-019-12901-3.
Pfeiffer, A., Thalheimer, F.B., Hartmann, S., Frank, A.M., Bender, R.R., Danisch, S., Costa, C., Wels, W.S., Modlich, U., Stripecke, R., Verhoeyen, E., Buchholz, C.J., 2018. In vivo generation of human CD19-CAR T cells results in B-cell depletion and signs of cytokine release syndrome. EMBO molecular medicine 10. https://doi.org/10.15252/emmm.201809158.
Pilipow, K., Scamardella, E., Puccio, S., Gautam, S., Paoli, F. de, Mazza, E.M., Simone, G. de, Polletti, S., Buccilli, M., Zanon, V., Di Lucia, P., Iannacone, M., Gattinoni, L., Lugli, E., 2018. Antioxidant metabolism regulates CD8+ T memory stem cell formation and antitumor immunity. JCI insight 3. https://doi.org/10.1172/jci.insight.122299.
Reschke, R., Yu, J., Flood, B., Higgs, E.F., Hatogai, K., Gajewski, T.F., 2021. Immune cell and tumor cell-derived CXCL10 is indicative of immunotherapy response in metastatic melanoma. Journal for immunotherapy of cancer 9. https://doi.org/10-1136/jitc-2021-003521.
Roberto, A., Castagna, L., Zanon, V., Bramanti, S., Crocchiolo, R., McLaren, J.E., Gandolfi, S., Tentorio, P., Sarina, B., Timofeeva, I., Santoro, A., Carlo-Stella, C., Bruno, B., Carniti, C., Corradini, P., Gostick, E., Ladell, K., Price, D.A., Roederer, M., Mavilio, D., Lugli, E., 2015. Role of naive-derived T memory stem cells in T-cell reconstitution following allogeneic transplantation. Blood 125, 2855-2864. https://doi.org/10.1182/blood-2014-11-608406.
Rosenberg, S.A., Restifo, N.P., 2015. Adoptive cell transfer as personalized immunotherapy for human cancer. Science (New York, N.Y.) 348, 62-68. https://doi.org/10.1126/science.aaa4967.
Roychoudhuri, R., Clever, D., Li, P., Wakabayashi, Y., Quinn, K.M., Klebanoff, C.A., Ji, Y., Sukumar, M., Eil, R.L., Yu, Z., Spolski, R., Palmer, D.C., Pan, J.H., Patel, S.J., Macallan, D.C., Fabozzi, G., Shih, H.-Y., Kanno, Y., Muto, A., Zhu, J., Gattinoni, L., O'Shea, J.J., Okkenhaug, K., Igarashi, K., Leonard, W.J., Restifo, N.P., 2016. BACH2 regulates CD8(+) T cell differentiation by controlling access of AP-1 factors to enhancers. Nature immunology 17, 851-860. https://doi.org/10.1038/ni.3441.
Ryu, D., Mouchiroud, L., Andreux, P.A., Katsyuba, E., Moullan, N., Nicolet-Dit-Félix, A.A., Williams, E.G., Jha, P., Lo Sasso, G., Huzard, D., Aebischer, P., Sandi, C., Rinsch, C., Auwerx, J., 2016. Urolithin A induces mitophagy and prolongs lifespan in C. elegans and increases muscle function in rodents. Nature medicine, 879-888. https://doi.org/10.1038/nm.4132.
Sato, T., Stange, D.E., Ferrante, M., Vries, R.G.J., van Es, J.H., van den Brink, S., van Houdt, W.J., Pronk, A., van Gorp, J., Siersema, P.D., Clevers, H., 2011. Long-term expansion of epithelial organoids from human colon, adenoma, adenocarcinoma, and Barrett's epithelium. Gastroenterology 141, 1762-1772. https://doi.org/10.1053/j.gastro.2011.07.050.
Scharping, N.E., Menk, A.V., Moreci, R.S., Whetstone, R.D., Dadey, R.E., Watkins, S.C., Ferris, R.L., Delgoffe, G.M., 2016. The Tumor Microenvironment Represses T Cell Mitochondrial Biogenesis to Drive Intratumoral T Cell Metabolic Insufficiency and Dysfunction. Immunity 45, 374-388. https://doi.org/10.1016/j.immuni.2016.07.009.
Schmitt, M., Greten, F.R., 2021. The inflammatory pathogenesis of colorectal cancer. Nature reviews. Immunology 21, 653-667. https://doi.org/10.1038/s41577-021-00534-x.
Schnalzger, T.E., Groot, M.H. de, Zhang, C., Mosa, M.H., Michels, B.E., Röder, J., Darvishi, T., Wels, W.S., Farin, H.F., 2019. 3D model for CAR-mediated cytotoxicity using patient-derived colorectal cancer organoids. The EMBO journal 38. https://doi.org/10.15252/embj.2018100928.
Scholz, G., Jandus, C., Zhang, L., Grandclément, C., Lopez-Mejia, I.C., Soneson, C., Delorenzi, M., Fajas, L., Held, W., Dormond, O., Romero, P., 2016. Modulation of mTOR Signalling Triggers the Formation of Stem Cell-like Memory T Cells. EBioMedicine 4, 50-61. https://doi.org/10.1016/j.ebiom.2016.01.019.
Schumann, J., Stanko, K., Schliesser, U., Appelt, C., Sawitzki, B., 2015. Differences in CD44 Surface Expression Levels and Function Discriminates IL-17 and IFN-γ Producing Helper T Cells. PloS one 10, e0132479. https://doi.org/10.1371/journal.pone.0132479.
Schwank, G., Clevers, H., 2016. CRISPR/Cas9-Mediated Genome Editing of Mouse Small Intestinal Organoids. Methods in molecular biology (Clifton, N.J.) 1422, 3-11. https://doi.org/10.1007/978-1-4939-3603-8_1.
Seeram, N.P., Adams, L.S., Henning, S.M., Niu, Y., Zhang, Y., Nair, M.G., Heber, D., 2005. In vitro antiproliferative, apoptotic and antioxidant activities of punicalagin, ellagic acid and a total pomegranate tannin extract are enhanced in combination with other polyphenols as found in pomegranate juice. The Journal of nutritional biochemistry 16, 360-367. https://doi.org/10.1016/j.jnutbio.2005.01.006.
Shen, P.-X., Li, X., Deng, S.-Y., Zhao, L., Zhang, Y.-Y., Deng, X., Han, B., Yu, J., Li, Y., Wang, Z.-Z., Zhang, Y., 2021. Urolithin A ameliorates experimental autoimmune encephalomyelitis by targeting aryl hydrocarbon receptor. EBioMedicine 64, 103227. https://doi.org/10.1016/j.ebiom.2021.103227.
Sung, H., Ferlay, J., Siegel, R.L., Laversanne, M., Soerjomataram, I., Jemal, A., Bray, F., 2021. Global Cancer Statistics 2020: GLOBOCAN Estimates of Incidence and Mortality Worldwide for 36 Cancers in 185 Countries. CA: a cancer journal for clinicians 71, 209-249. https://doi.org/10.3322/caac.2166o.
Toney, A.M., Fox, D., Chaidez, V., Ramer-Tait, A.E., Chung, S., 2021. Immunomodulatory Role of Urolithin A on Metabolic Diseases. Biomedicines 9. https://doi.org/10.3390/biomedicines9020192.
Totiger, T.M., Srinivasan, S., Jala, V.R., Lamichhane, P., Dosch, A.R., Gaidarski, A.A., Joshi, C., Rangappa, S., Castellanos, J., Vemula, P.K., Chen, X., Kwon, D., Kashikar, N., VanSaun, M., Merchant, N.B., Nagathihalli, N.S., 2019. Urolithin A, a Novel Natural Compound to Target PI3K/AKT/mTOR Pathway in Pancreatic Cancer. Molecular cancer therapeutics, 301-311. https://doi.org/10.1158/1535-7163.MCT-18-0464.
Utzschneider, D.T., Charmoy, M., Chennupati, V., Pousse, L., Ferreira, D.P., Calderon-Copete, S., Danilo, M., Alfei, F., Hofmann, M., Wieland, D., Pradervand, S., Thimme, R., Zehn, D., Held, W., 2016. T Cell Factor 1-Expressing Memory-like CD8(+) T Cells Sustain the Immune Response to Chronic Viral Infections. Immunity 45, 415-427. https://doi.org/10.1016/j.immuni.2016.07.021.
van de Wetering, M., Francies, H.E., Francis, J.M., Bounova, G., Iorio, F., Pronk, A., van Houdt, W., van Gorp, J., Taylor-Weiner, A., Kester, L., McLaren-Douglas, A., Blokker, J., Jaksani, S., Bartfeld, S., Volckman, R., van Sluis, P., Li, V.S.W., Seepo, S., Sekhar Pedamallu, C., Cibulskis, K., Carter, S.L., McKenna, A., Lawrence, M.S., Lichtenstein, L., Stewart, C., Koster, J., Versteeg, R., van Oudenaarden, A., Saez-Rodriguez, J., Vries, R.G.J., Getz, G., Wessels, L., Stratton, M.R., McDermott, U., Meyerson, M., Garnett, M.J., Clevers, H., 2015. Prospective derivation of a living organoid biobank of colorectal cancer patients. Cell 161, 933-945. https://doi.org/10.1016/j.cell.2015.03.053.
van den Eynde, M., Mlecnik, B., Bindea, G., Fredriksen, T., Church, S.E., Lafontaine, L., Haicheur, N., Marliot, F., Angelova, M., Vasaturo, A., Bruni, D., Jouret-Mourin, A., Baldin, P., Huyghe, N., Haustermans, K., Debucquoy, A., van Cutsem, E., Gigot, J.-F., Hubert, C., Kartheuser, A., Remue, C., Léonard, D., Valge-Archer, V., Pages, F., Machiels, J.-P., Galon, J., 2018. The Link between the Multiverse of Immune Microenvironments in Metastases and the Survival of Colorectal Cancer Patients. Cancer cell 34, 1012-1026.e3. https://doi.org/10.1016/j.ccell.2018.11.003.
Vannini, N., Campos, V., Girotra, M., Trachsel, V., Rojas-Sutterlin, S., Tratwal, J., Ragusa, S., Stefanidis, E., Ryu, D., Rainer, P.Y., Nikitin, G., Giger, S., Li, T.Y., Semilietof, A., Oggier, A., Yersin, Y., Tauzin, L., Pirinen, E., Cheng, W.-C., Ratajczak, J., Canto, C., Ehrbar, M., Sizzano, F., Petrova, T.V., Vanhecke, D., Zhang, L., Romero, P., Nahimana, A., Cherix, S., Duchosal, M.A., Ho, P.-C., Deplancke, B., Coukos, G., Auwerx, J., Lutolf, M.P., Naveiras, O., 2019. The NAD-Booster Nicotinamide Riboside Potently Stimulates Hematopoiesis through Increased Mitochondrial Clearance. Cell stem cell 24, 405-418.e7. https://doi.org/10.1016/j.stem.2019.02.012.
Varga, J., Nicolas, A., Petrocelli, V., Pesic, M., Mahmoud, A., Michels, B.E., Etlioglu, E., Yepes, D., Häupl, B., Ziegler, P.K., Bankov, K., Wild, P.J., Wanninger, S., Medyouf, H., Farin, H.F., Tejpar, S., Oellerich, T., Ruland, J., Siebel, C.W., Greten, F.R., 2020. AKT-dependent NOTCH3 activation drives tumor progression in a model of mesenchymal colorectal cancer. The Journal of experimental medicine 217. https://doi.org/10.1084/jem.20191515.
Verma, V., Jafarzadeh, N., Boi, S., Kundu, S., Jiang, Z., Fan, Y., Lopez, J., Nandre, R., Zeng, P., Alolaqi, F., Ahmad, S., Gaur, P., Barry, S.T., Valge-Archer, V.E., Smith, P.D., Banchereau, J., Mkrtichyan, M., Youngblood, B., Rodriguez, P.C., Gupta, S., Khleif, S.N., 2021. MEK inhibition reprograms CD8+ T lymphocytes into memory stem cells with potent antitumor effects. Nature immunology 22, 53-66. https://doi.org/10.1038/s41590-020-00818-9.
Weidner, T., Agarwal, S., Perian, S., Fusil, F., Braun, G., Hartmann, J., Verhoeyen, E., Buchholz, C.J., 2021. Genetic in vivo engineering of human T lymphocytes in mouse models. Nature protocols 16, 3210-3240. https://doi.org/10.1038/s41596-021-00510-8.
Xiong, Y., Wang, L., Di Giorgio, E., Akimova, T., Beier, U.H., Han, R., Trevisanut, M., Kalin, J.H., Cole, P.A., Hancock, W.W., 2020. Inhibiting the coregulator CoREST impairs Foxp3+ Treg function and promotes antitumor immunity. The Journal of clinical investigation 130, 1830-1842. https://doi.org/10.1172/JCI131375.
Yamaguchi, A., Ishikawa, H., Furuoka, M., Yokozeki, M., Matsuda, N., Tanimura, S., Takeda, K., 2019. Cleaved PGAM5 is released from mitochondria depending on proteasome-mediated rupture of the outer mitochondrial membrane during mitophagy. Journal of biochemistry 165, 19-25. https://doi.org/10.1093/jb/mvy077.
Yu, B., Ma, J., Li, J., Wang, D., Wang, Z., Wang, S., 2020a. Mitochondrial phosphatase PGAM5 modulates cellular senescence by regulating mitochondrial dynamics. Nature communications 11, 2549. https://doi.org/10.1038/s41467-020-16312-7.
Yu, Y.-R., Imrichova, H., Wang, H., Chao, T., Xiao, Z., Gao, M., Rincon-Restrepo, M., Franco, F., Genolet, R., Cheng, W.-C., Jandus, C., Coukos, G., Jiang, Y.-F., Locasale, J.W., Zippelius, A., Liu, P.-S., Tang, L., Bock, C., Vannini, N., Ho, P.-C., 2020b. Disturbed mitochondrial dynamics in CD8+ TILs reinforce T cell exhaustion. Nature immunology 21, 1540-1551. https://doi.org/10.1038/s41590-020-0793-3.
Zhou, X., Yu, S., Zhao, D.-M., Harty, J.T., Badovinac, V.P., Xue, H.-H., 2010. Differentiation and persistence of memory CD8(+) T cells depend on T cell factor 1. Immunity 33, 229-240. https://doi.org/10.1016/j.immuni.2010.08.002.
Ziegler, P.K., Bollrath, J., Pallangyo, C.K., Matsutani, T., Canli, Ö., Oliveira, T. de, Diamanti, M.A., Müller, N., Gamrekelashvili, J., Putoczki, T., Horst, D., Mankan, A.K., Öner, M.G., Müller, S., Müller-Höcker, J., Kirchner, T., Slotta-Huspenina, J., Taketo, M.M., Reinheckel, T., Dröse, S., Larner, A.C., Wels, W.S., Ernst, M., Greten, T.F., Arkan, M.C., Korn, T., Wirth, D., Greten, F.R., 2018. Mitophagy in Intestinal Epithelial Cells Triggers Adaptive Immunity during Tumorigenesis. Cell 174, 88-101.e16. https://doi.org/10.1016/j.cell.2018.05.028.

## Claims

1. **A compound for use in a therapeutic modulation of a T-cell mediated immune response** in a subject, wherein the compound is a mitophagy agonist, preferably selected from a compound of formula (I), 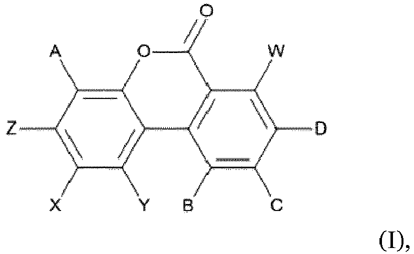 wherein A, B, C, D, X, Y, and W are independently selected from H or OH, or a salt, isomer, tautomer, prodrug thereof.

2. The compound for use of claim 1, wherein the compound is selected from urolithin A, urolithin B, urolithin C and urolithin D.

3. The compound for use of any one of claims 1 or 2, wherein the modulation of a T-cell mediated immune response involves any one or a combination of:
(i) an induction of mitophagy in the T-cell;
(ii) an increase in T-memory stem cells (T_{SCM});
(iii) an increase in naïve like T-cells (T_{N});
(iv) an increase in T-cell mediated anti-tumor immunity;
(v) increase in T-cell WNT signaling;
(vi) increased T-cell mitochondrial health.

4. The compound for use of any one of claims 1 to 3, wherein the use comprises an administration of the compound to the subject, or comprises an administration of T-cells contacted with the compound to the subject, preferably wherein the T-cells contacted with the compound are autologous T-cells obtained from the subject, subsequently brought into contact with the compound, optionally, expanded and/or genetically modified, and reinfused to the patient.

5. **An *ex-vivo* method for modulating a T-cell,** the method comprising:
**a.** Providing a T-cell;
**b.** Contacting the T-cell with an mitophagy agonist, preferably a compound of formula (I), 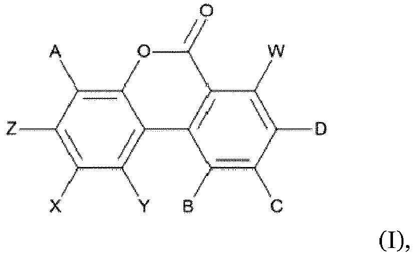 wherein A, B, C, D, X, Y, and W are independently selected from H or OH, or a salt, isomer, tautomer, or prodrug thereof,
for a time sufficient to induce mitophagy in the T-cell, and thereby obtain a modulated T-cell;
**c.** Optionally, further genetically modifying the modulated T-cell;
**d.** Optionally, further culturing and / or proliferating the modulated T-cell.

6. The method of claim 5, wherein in step **b** the T-cell is modulated into a T-memory stem cells (T_{SCM}) or a naïve like T-cell (T_{N});

7. The method of claim 5 or 6, wherein step **c** includes genetically introducing a construct, such as a Chimeric Antigen Receptor (CAR), for enhancing an immune response mediated by the T-cell.

8. **A T-cell** obtainable by, or obtained by, a method of any one of claims 1 to 7.

9. **A cellular composition,** comprising multiple T-cells according to claim 8.

10. **A cellular composition for use in the treatment of a disease** in a subject, wherein the treatment comprises an adoptive T-cell transfer with the steps of
**a.** Obtaining a cellular sample of the subject containing T-cells;
**b.** Optionally purifying and/or culturing (expanding) the T-cells from the cellular sample;
**c.** Contacting the T-cell with an mitophagy agonist, preferably a compound of formula (I), 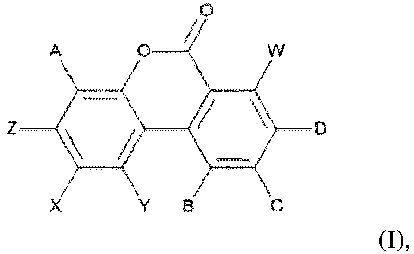 wherein A, B, C, D, X, Y, and W are independently selected from H or OH, or a salt, isomer, tautomer, or prodrug thereof,
for a time sufficient to induce mitophagy in the T-cell, and thereby obtain modulated T-cells;
**d.** Optionally, further genetically modifying the modulated T-cells;
**e.** Optionally, further culturing and / or proliferating the modulated T-cells.
**f.** Administrating the modulated T-cells to the patient and thereby treating the disease.

11. The cellular composition for use of claim 10 wherein in step **c** the T-cell is modulated into a T-memory stem cells (T_{SCM}) or a naïve like T-cell (T_{N}).

12. The cellular composition for use of claims 10 or 11, wherein step **d** includes genetically introducing a construct, such as a CAR, for enhancing an immune response mediated by the T-cell.

13. The cellular composition for use of any one of claims 10 to 12, wherein the T-cell is provided from a cellular sample of a subject suffering from a disease treatable by T-cell activation, such as a proliferative disease (cancer) or an infectious disease.

14. The cellular composition for use of any one of claims 10 to 13, wherein the T-cell after modification is proliferated and expanded to obtain a clinical grade T-cell preparation suitable for using in adoptive T-cell therapy.

15. **A method for modulating a T-cell,** the method comprising the steps of contacting a T-cell with a mitophagy agonist for a time sufficient to induce mitophagy in the T-cell, preferably wherein the mitophagy agonist is a compound recited in claim 1.
